# EUROPÄISCHE PATENTSCHRIFT

(11) **EP 4 021 543 B1**
(45) Veröffentlichungstag und Bekanntmachung des Hinweises auf die Patenterteilung: **17.05.2023**
(21) Anmeldenummer: 20810941.3
(22) Anmeldetag: 19.11.2020
(51) Int. Cl.: A61M 16/04, A61M 16/08

(54) **ANSCHLUSSBAUTEIL FÜR EINE TRACHEALKANÜLE, INSBESONDERE ZUM SCHUTZ VOR UNBEABSICHTIGTEM VERSCHLIESSEN DERSELBEN**
CONNECTION COMPONENT FOR A TRACHEAL CANNULA, IN PARTICULAR FOR PROTECTING AGAINST AN UNINTENTIONAL CLOSURE OF SAME
COMPOSANT DE RACCORDEMENT POUR UNE CANULE TRACHÉALE, PLUS PARTICULIÈREMENT POUR LA PROTECTION CONTRE UNE FERMETURE INVOLONTAIRE DE CELLE-CI

(30) Priorität: 21.11.2019 DE 102019131549
(43) Veröffentlichungstag der Anmeldung: 06.07.2022
(73) Patentinhaber: Hamilton Medical AG, 7402 Bonaduz (CH)
(72) Erfinder: SUCCETTI, Martin, 7412 Scharans (CH)
(74) Vertreter: Ruttensperger Lachnit Trossin Gomoll
(86) Internationale Anmeldenummer: PCT/EP2020/082693
(87) Internationale Veröffentlichungsnummer: WO 2021/099470

(56) Entgegenhaltungen:
- EP-A1- 1 479 405
- DE-A1- 10 140 292
- DE-A1-102015 105 496
- DE-B3-102005 030 300
- US-A- 4 802 474
- US-A- 5 067 496
- US-A1- 2004 177 851
- US-A1- 2007 181 132
- US-A1- 2017 049 982

## Beschreibung

Die vorliegende Erfindung betrifft ein Anschlussbauteil für eine Trachealkanüle gemäß dem Oberbegriff des Anspruchs 1. Ein solches Anschlussbauteil umgibt unter anderem einen Hohlraum, wobei das Anschlussbauteil sowohl an einem Kopplungsbereich des Anschlussbauteils als auch an einem vom Kopplungsbereich örtlich verschiedenen Mündungsbereich des Anschlussbauteils zur Bereitstellung eines Zugangs zu dem Hohlraum offen oder öffenbar ist, wobei das Anschlussbauteil am Kopplungsbereich eine Verrastungsformation aufweist, welche für einen formschlüssigen Verrastungseingriff mit einer Verrastungsgegenformation der Trachealkanüle ausgebildet ist, wobei der Kopplungsbereich einen Abschnitt des Hohlraums umgibt, wobei der von dem Kopplungsbereich umgebene Abschnitt des Hohlraums zentral von einer virtuellen Kopplungsachse durchsetzt gedacht ist, wobei die Kopplungsachse längs ihres Verlaufs eine axiale Richtung, orthogonal zu ihrem Verlauf eine Vielzahl von radialen Richtungen und um sie umlaufend eine Umfangsrichtung definiert, wobei der Kopplungsbereich wenigstens einen Tragabschnitt aufweist, an welchem an einem ersten Ort die Verrastungsformation und an einem vom ersten Ort verschiedenen zweiten Ort ein Einwirkbereich derart angeordnet sind, dass durch Ausübung einer Betätigungskraft auf den Einwirkbereich in radialer Richtung auf die Kopplungsachse zu der Tragabschnitt verlagerbar ist zwischen zwei Stellungen unterschiedlichen radialen Abstands der am Tragabschnitt angeordneten Verrastungsformation von der Kopplungsachse.

Die vorliegende Erfindung betrifft weiter eine Beatmungsbaugruppe, umfassend eine Trachealkanüle oder wenigstens ein Trachealkanülen-Endstück und ein solches mit dem Trachealkanülen-Endstück koppelbares Anschlussbauteil.

Ein Anschlussbauteil der eingangs genannten Art und ebenso eine Beatmungsbaugruppe mit einem solchen Anschlussbauteil und einem Trachealkanülen-Endstück sind aus der US 2017/0049982 A1 bekannt.

Das bekannte Anschlussbauteil weist einen Kopplungsbereich auf, mit sich diametral bezüglich der Kopplungsachse gegenüberliegenden, in Umfangsrichtung um einen Teilbereich des Umfangs verlaufenden Schlitzen als Verrastungsformationen, welche zum Formschlusseingriff mit entsprechend ausgebildeten Vorsprüngen am Trachealkanülen-Endstück ausgebildet sind. Der Kopplungsbereich bildet dabei einen Endbereich des Anschlussbauteils. Ein in axialer Richtung bezüglich der Kopplungsachse gegenüberliegender Endbereich des bekannten Anschlussbauteils ist geschlossen und als abgerundete Kuppe ausgebildet.

In einem Zwischenbereich zwischen dem Kopplungsbereich und dem axial entgegengesetzten kuppelartigen Endbereich sind im Umfang des Anschlussbauteils, wiederum einander bezüglich der Kopplungsachse gegenüberliegend, Mündungsöffnungen ausgebildet, durch welche hindurch der vom Anschlussbauteil eingefasste Hohlraum zugänglich ist.

Im Umfangsbereich zwischen den Verrastungsformationen des bekannten Anschlussbauteils sind an dessen Außenfläche Einwirkbereiche mit Antirutsch-Rippen ausgebildet, die einen drückenden Fingerangriff zwischen den Verrastungsformationen erleichtern sollen, um durch Druck von außen auf die Antirutsch-Rippen in radialer Richtung auf die Kopplungsachse zu eine Verformung des Kopplungsbereichs zu erreichen und so das Anschlussbauteil am Trachealkanülen-Endstück anbringen und von diesem lösen zu können.

Der Kopplungsbereich hat im von außen unbelasteten Zustand einen ovalen Querschnitt bei Betrachtung eines Schnitts in einer zur Kopplungsachse orthogonalen Schnittebene. Die Einwirkbereiche liegen dabei - im unbelasteten Zustand des Anschlussbauteils - im Bereich der langen Halbachse des ovalen Querschnitts, die Verrastungsformationen im Bereich der kurzen Halbachsen. Durch Druck von außen in Richtung zur Kopplungsachse hin kann der Kopplungsbereich in eine Gestalt mit kreisförmigem Querschnitt verformt werden und in diesen Verformungszustand über einen im Wesentlichen zylindrischen Gegenkopplungsbereich des Trachealkanülen-Endstück geschoben werden. Endet die Ausübung des verformenden Drucks auf die Einwirkbereiche, wirken elastische Rückstellkräfte, welche den Kopplungsbereich zurück in die ursprüngliche ovale Querschnittsgestalt drängen. An der Rückstellung werden sie jedoch vor allem im Bereich der kurzen Halbachsen durch den axial mit dem Kopplungsbereich überlappenden Gegenkopplungsbereich gehindert, was grundsätzlich geeignet ist, die Verbindung zwischen Anschlussbauteil und Trachealkanülen-Endstück mit höherer Sicherheit gegen unerwünschtes Lösen des Anschlussbauteils vom Endstück auszubilden.

Ein weiteres Anschlussbauteil ist aus der US 5520174 A bekannt. Dieses bekannte Anschlussbauteil wird lediglich mit seinem Kopplungsbereich auf einen Gegenkopplungsbereich einer Trachealkanüle aufgeschoben und dort reibschlüssig gehalten.

Aus der US 2007/0181132 A1 sind ein Trachealkanülen-Endstück und ein mit dem Trachealkanülen-Endstück verrastbares Anschlussbauteil bekannt. Seitens des Anschlussbauteils ist eine Rastklaue an einem Längsende eines ausschließlich axial längs des Anschlussbauteils verlaufenden Rasthebels derart ausgebildet, dass durch Betätigung des Rasthebels an seinem der Rastklaue axial entgegengesetzten Ende die Rastklaue außer Eingriff mit einer Eingriffsformation am Trachealkanülen-Endstück bewegt werden kann.

Aus der EP 1 479 405 A1 ist ein weiteres an einem Trachealkanülen-Endstück verrastbares Anschlussbauteil bekannt. Die nach radial innen vorstehenden Rastnasen sind an einem hohlzylindrischen Rastbauteil an diametral einander gegenüberliegenden Umfangsabschnitten ausgebildet. In Umfangsrichtung zwischen den Rastnasen gelegene Umfangsabschnitte des hohlzylindrischen Rastbauteils sind frei von Rastnasen, so dass diese Umfangsabschnitte nach radial innen verformt werden können, um die Verrastung zu lösen. Diese Verformung bewirkt eine Verlagerung der Rastnasen nach radial außen und damit ein Lösen der Verrastung.

Die US 5067496 offenbart ein mehrteiliges Tracheotomierohr. Ein radial inneres Tracheotomierohr-Bauteil kann in ein radial äußeres Tracheotomierohr-Bauteil axial eingeführt werden. Ein Endbereich des inneren Tracheotomierohr-Bauteils ist zu groß, um in das äußere Tracheotomierohr-Bauteil eingeführt zu werden und verbleibt daher axial außerhalb des äußeren Tracheotomierohr-Bauteils. Der Endbereich kann an einer nach radial außen starr vorstehenden Rastkante des äußeren Tracheotomierohr-Bauteils verrastet werden. Am Endbereich ist hierzu eine die Rastkante im Rasteingriff hintergreifende Rastklaue an einem Längsende eines ausschließlich axial längs des Anschlussbauteils verlaufenden Rasthebels ausgebildet. Durch Betätigung des Rasthebels an seinem der Rastklaue axial entgegengesetzten Ende kann die Rastklaue außer Eingriff mit der Rastkante bewegt werden.

Aus der DE 101 40 292 A1 sind ein Trachealkanülen-Endstück und ein an dem Trachealkanülen-Endstück durch eine Bajonett-Verriegelung festlegbares Anschlussbauteil bekannt.

Für weiteren, jedoch technisch ferner ab liegenden Stand der Technik zu Trachealkanülen und damit gekoppelte Bauteile wird auf die DE 10 2015 105 496 A1 und auf die DE 10 2005 030 300 B3 verwiesen.

Von der vorliegenden Anmeldung betroffene Anschlussbauteile dienen zumeist dazu, ein unerwünschtes Verschließen einer Trachealkanüle durch Körperteile des die Trachealkanüle tragenden Patienten zu vermeiden. Insbesondere die Kinnpartie eines solchen Patienten kann das offene Ende einer Trachealkanüle erreichen und bei ungünstiger Lage verschließen, sodass eine Strömung von Atemgas durch die Trachealkanüle verhindert würde. Dies kann für den Patienten fatale Folgen haben.

Aufgrund dieser Schutzfunktion sind von der vorliegenden Anmeldung betroffene Anschlussbauteile im einschlägigen Fachgebiet auch im deutschen Sprachgebiet als "tracheostomy guard", "tracheotomy guard", ""trach guard" oder "tracheostomy tube guard" bezeichnet.

Nachteilig an dem aus der US 2017/0049982 A1 bekannten Anschlussbauteil ist unter anderem sein Lösevorgang, da im auf den Gegenkopplungsbereich der Trachealkanüle aufgesetzten Zustand eine den Kopplungsbereich des Anschlussbauteils verformende Krafteinwirkung auf den Einwirkbereich durch den Gegenkopplungsbereich der Trachealkanüle behindert ist. Als Folge kann es notwendig sein, eine sehr hohe verformende Kraft auszuüben, die allerdings nur schwer zu kontrollieren ist, da die Trachealkanüle in den Patienten eingeführt und daher nicht beliebig manipulierbar ist. Somit kann der Lösevorgang über die Trachealkanüle als Kraftvermittlerin eine unerwünschte Kraftausübung auf den Patienten zur Folge haben.

Es ist Aufgabe der vorliegenden Erfindung, das eingangs genannte Anschlussbauteil derart weiterzubilden, dass es mit hoher Patientenfreundlichkeit, also mit möglichst geringer Belastung des eine Trachealkanüle tragenden Patienten, mit der Trachealkanüle verbunden und von dieser gelöst werden kann.

Diese Aufgabe löst die vorliegende Erfindung durch ein Anschlussbauteil mit allen Merkmalen des Anspruchs 1.

Das Risiko einer Verletzung des Patienten kann durch das Anschlussbauteil durch Vermeidung von Ecken und Kanten an der Außenfläche des Anschlussbauteils verringert werden. Zu diesem Zweck ist das Anschlussbauteil erfindungsgemäß zwischen dem Kopplungsbereich und dem Mündungsbereich als um eine zur Kopplungsachse orthogonale Krümmungsachse gekrümmtes Rohr oder Hutze ausgebildet. Der Mündungsbereich kann dann vom Kopf des Patienten wegweisend mit dessen Trachealkanüle verbunden werden, sodass ein kantenfreier, gerundeter Abschnitt des Anschlussbauteils zum Patienten, insbesondere zu dessen Kinnpartie, hinweist.

Durch die axial beabstandete Anordnung von Einwirkbereich und Verrastungsformation ist es gemäß der vorliegenden Erfindung anders als bei der oben beschriebenen US 2017/0049982 A1 außerdem möglich, nicht den gesamten Kopplungsbereich durch eine radial auf die Kopplungsachse zu auf den Einwirkbereich ausgeübte Kraft zu verformen, sondern nur den Tragabschnitt. In der Folge kann der Einwirkbereich wegen des Axialabschnitts zur Verrastungsformation in einem Bereich des Anschlussbauteils gelegen sein, welcher in einem an die Trachealkanüle bestimmungsgemäß angebrachten Zustand nicht mit Bauteilen der Trachealkanüle überlappt. Folglich kann der Einwirkbereich ungehindert durch Bauteile oder Bauteilabschnitte der Trachealkanüle mit Kraft beaufschlagt und verlagert werden. Eine Verlagerung der Verrastungsformation, insbesondere zur Aufhebung eines Formschlusseingriffs mit einer Verrastungsgegenformation der Trachealkanüle, kann somit aus zwei Gründen durch Ausübung einer betragsmäßig geringeren Kraft auf den Einwirkbereich bewirkt werden als im Stand der Technik: der zu verformende Bauteilbereich ist kleiner und eine Verlagerung des Einwirkbereichs ist auch im gekoppelten Zustand nicht durch die Trachealkanüle behindert oder erschwert. Eine geringere Kraft lässt sich leichter exakt dosieren und applizieren..

Der vom Anschlussbauteil umgebene Hohlraum ist vorzugsweise ein zusammenhängender Hohlraum, sodass der Hohlraum den Kopplungsbereich mit dem Mündungsbereich verbindet. Der Hohlraum bildet somit bevorzugt also einen Strömungskanal zwischen Kopplungsbereich und Mündungsbereich, längs welchem Atemgas zwischen Kopplungsbereich und Mündungsbereich strömen kann. Einer der Bereiche oder beide Bereiche können aus hygienischen Gründen vor einer Kopplung mit einer Trachealkanüle durch Verschlussmittel verschlossen sein. Jedoch sind diese Verschlussmittel vom jeweiligen Bereich abnehmbar, sodass am Kopplungsbereich eine Kopplungsöffnung und am Mündungsbereich eine Mündungsöffnung vorliegen, durch welche hindurch der vom Anschlussbauteil umgebene Hohlraum von der Außenumgebung des Anschlussbauteils aus erreichbar ist.

Um einen möglichst großen Anteil der auf den Einwirkbereich in radialer Richtung auf die Kopplungsachse zu ausgeübten Kraft für eine Verlagerung der am selben Tragabschnitt wie der Einwirkbereich angeordneten Verrastungsformation nutzen zu können, ist es vorteilhaft, wenn der Einwirkbereich und die Verrastungsformation desselben Tragabschnitts in Umfangsrichtung um die Kopplungsachse wenigstens abschnittsweise im gleichen Umfangsbereich angeordnet sind. Noch stärker bevorzugt sind Einwirkbereich und Verrastungsformation vollständig im gleichen Umfangsbereich angeordnet, sodass zwischen Einwirkbereich und Verrastungsformation desselben Tragabschnitts nur ein axialer Abstand, jedoch kein Umfangsabstand besteht.

Zur Klarstellung seien ein Einwirkbereich und eine Verrastungsformation dann abschnittsweise im gleichen Umfangsbereich angeordnet, wenn der Einwirkbereich die Verrastungsformation in einer ersten Umfangsrichtung überragt und die Verrastungsformation den Einwirkbereich in einer der ersten entgegengesetzten zweiten Umfangsrichtung überragt. Überragt ein Bauteilabschnitt aus Einwirkbereich und Verrastungsformation den jeweils anderen in beiden entgegengesetzten Umfangsrichtungen, weil der eine eine größere Umfangserstreckung aufweist als der jeweils andere, oder nur in einer Umfangsrichtung, ohne dass der andere den einen in der entgegengesetzten Umfangsrichtung überragt, oder überragt kein Bauteilabschnitt den jeweils anderen, weil sie eine gleiche Umfangserstreckung aufweisen, dann sind Einwirkbereich und Verrastungsformation vollständig im gleichen Umfangsbereich angeordnet.

In einer bevorzugten konstruktiven Ausgestaltung kann der Tragabschnitt einen mit einem Bauteilhauptkörper relativ zu diesem beweglich verbundenen Tragarm umfassen. Durch Ausübung der Betätigungskraft kann der Tragarm bezüglich des Bauteilhauptkörpers um eine zur Kopplungsachse windschiefe Neigeachse neigbar sein, um dadurch die Verlagerung der Formschlussformation unter Annäherung an die oder/und unter Entfernung von der Kopplungsachse zu bewirken. "Windschief" bedeutet dabei mit Abstand von der Kopplungsachse und nicht-parallel zu dieser. Für eine besonders effektive Verlagerung der Verrastungsformation auf die Kopplungsachse zu und von dieser weg schließt die Erstreckungsrichtung der Neigeachse mit der Erstreckungsrichtung der Kopplungsachse bevorzugt einen Winkel im Bereich von 70° bis 110° ein. Da der Abstand zwischen Einwirkbereich und Verrastungsformation axial, also längs der Kopplungsachse verläuft, schließt die Neigeachse mit der Kopplungsachse bevorzugt einen rechten Winkel ein.

Der Tragabschnitt kann als einarmiger Hebel ausgebildet sein, wobei dann der Einwirkbereich und die Verrastungsformation auf derselben Seite der Neigeachse am selben Tragabschnitt gelegen sind, sodass eine radiale Betätigungsverlagerung des Einwirkbereichs eine gleichsinnige, aber betragsmäßig von der Betätigungsverlagerung verschiedene Wirkverlagerung der Verrastungsformation desselben Tragabschnitts bewirkt. Eine betragsmäßig geringere Verlagerung des Einwirkbereichs radial zur Kopplungsachse hin kann so eine betragsmäßig größere Verlagerung der axial vom Einwirkbereich entfernt gelegenen Verrastungsformation bewirken. Hierzu kann der Einwirkbereich näher an der Neigeachse gelegen sein als die durch Anordnung am selben Tragabschnitt zugeordnete Verrastungsformation. Der Tragabschnitt übersetzt dann die Betätigungsverlagerung in eine betragsmäßig größere Wirkverlagerung.

Alternativ zu der zuvor genannten Lösung kann der Tragabschnitt als zweiarmiger Hebel ausgebildet sein, wobei je ein Hebel in einer anderen, vorzugsweise diametral entgegengesetzten, Richtung von der Neigeachse absteht. In diesem Falle sind der Einwirkbereich und die Verrastungsformation auf unterschiedlichen Seiten der Neigeachse am selben Tragabschnitt gelegen, sodass eine radiale Betätigungsverlagerung des Einwirkbereichs eine gegensinnige Wirkverlagerung der Verrastungsformation desselben Tragabschnitts bewirkt.

Unabhängig von einer ein- oder zweiarmigen Ausgestaltung ist der wenigstens eine Tragabschnitt bevorzugt derart am Anschlussbauteil ausgebildet, dass der Einwirkbereich nur in eine Richtung, nämlich radial auf die Kopplungsachse zu, betätigt wird und hierdurch eine Verlagerung der Verrastungsformation bewirkt. Nach dem Ende einer Kraftausübung auf den Einwirkbereich kann eine Rückstellung der Verrastungsformation aufgrund von am Anschlussbauteil vorgesehenen Rückstellmitteln, einschließlich der Materialelastizität der an der Verlagerung des Tragabschnitts beteiligten Bauteilabschnitte, erfolgen. Die Betätigung des Einwirkbereichs dient bevorzugt dem Lösen eines Formschlusseingriffs zwischen Verrastungsformation und Verrastungsgegenformation, nicht der Herstellung eines solchen Formschlusseingriffs. Dieser kann dauerhaft durch die oben beschriebenen Rückstellmittel sichergestellt sein, die einer Verlagerung der Verrastungsformation aus ihrem Formschlusseingriff heraus entgegenwirken. Die Herstellung eines formschlüssigen Verrastungseingriffs von Verrastungsformation und Verrastungsgegenformation kann unter der Vorspannwirkung der Rückstellmittel selbsttätig erfolgen.

Somit kann durch die oben beschriebene Lösung des Tragabschnitts als einarmiger Hebel ein Verrastungseingriff der Verrastungsformation mit der Verrastungsgegenformation ein Formschlusseingriff sein, bei welchem der Tragabschnitt oder/und die Verrastungsformation bezogen auf die Kopplungsachse radial innerhalb des Gegenkopplungsbereichs oder/und der Verrastungsgegenformation der Trachealkanüle angeordnet ist.

Sofern nach Herstellung eines Verrastungseingriffs zwischen Anschlussbauteil und Trachealkanüle eine Anordnung des Tragabschnitts oder/und der Verrastungsformation radial außerhalb des Gegenkopplungsbereichs oder/und der Verrastungsgegenformation der Trachealkanüle gewünscht ist, kann dies durch die oben beschriebene Lösung des Tragabschnitts als zweiarmiger Hebel realisiert werden.

Grundsätzlich kann daran gedacht sein, den Tragarm mittels eines gesonderten Achsbauteils um die Neigeachse beweglich am Bauteilhauptkörper anzuordnen. Zusätzlich kann ein Federbauteil als ein Vorspann- oder/und Rückstellmittel vorgesehen sein, um den Tragarm in eine vorbestimmte Stellung vorzuspannen, aus welcher er durch die oben beschriebene Krafteinwirkung auf den Einwirkbereich ausgelenkt werden kann. Eine solche aus mehreren Bauteilen gebaut Lösung ist jedoch aufwendig. Aufgrund der resultierenden geringeren Bauteileanzahl ist daher bevorzugt, dass der Tragarm einstückig mit dem Bauteilhauptkörper verbunden ist, so dass eine Verlagerung der Verrastungsformation durch Verformung wenigstens eines Abschnitts des Anschlussbauteils bewirkt ist. In diesem Falle kann die Neigeachse eine durch Material des Tragarms und des Bauteilhauptkörpers gebildete Biegeachse oder/und Torsionsachse sein. Die Neigeachse wird überwiegend oder vollständig eine Biegeachse im oben genannten Fall der Ausbildung des Tragabschnitts als einarmiger Hebel sein, dessen Tragarm einstückig mit dem Bauteilhauptkörper verbunden ist. Die Neigeachse wird überwiegend oder vollständig eine Torsionsachse im oben genannten Fall der Ausbildung des Tragabschnitts als zweiarmiger Hebel sein, dessen Tragarm einstückig mit dem Bauteilhauptkörper verbunden ist.

Der Tragarm und der Bauteilhauptkörper können einfach und kostengünstig mit hoher Präzision in den Abmessungen als Kunststoff-Spritzgussbauteil hergestellt sein.

Durch die einstückige Ausbildung des Tragarms mit dem Bauteilhauptkörper kann außerdem bei entsprechender Bauteildimensionierung die Bauteilelastizität zur Bereitstellung einer rückstellenden Kraft nach dem Ende einer Krafteinwirkung auf die Einwirkbereiche genutzt werden. Somit ist das Anschlussbauteil selbst sein eigenes Vorspann- und Rückstellmittel. Ein gesondertes Federbauteil zur Rückstellung einer Verrastungsformation wird dann nicht benötigt.

Zur verbesserten Lagedefinition des Anschlussbauteils dann, wenn es bestimmungsgemäß mit einer Trachealkanüle verbunden ist, kann der Kopplungsbereich eine Schürze aufweisen, welche die Kopplungsachse längs eines Winkelbereichs umgibt. Zur Sicherstellung der Beweglichkeit des Tragabschnitts relativ zu der erwähnten Schürze kann der Tragabschnitt von der Schürze durch wenigstens eine Nut getrennt sein. Die Schürze kann dann, wenn die Trachealkanüle mit dem Anschlussbauteil bestimmungsgemäß gekoppelt ist, einer entsprechenden Gegenschürze des Gegenkopplungsbereichs mit geringem Radialspalt flächig gegenüberliegen, sodass der so gebildete Radialspalt eine Relativbeweglichkeit von Anschlussbauteil und Trachealkanüle begrenzt, vor allem eine Kippbewegung um eine zur Kopplungsachse orthogonale Kippachse. Die wenigstens Nut durchsetzt bevorzugt das Anschlussbauteil in Dickenrichtung vollständig. Der Tragabschnitt kann zur Sicherstellung seiner Beweglichkeit zur Kopplungsachse hin und von dieser weg wenigstens in einem die Verrastungsformation enthaltenen Bereich zwischen zwei Nuten angeordnet sein, von welchen vorzugsweise beide das Anschlussbauteil in Dickenrichtung vollständig durchsetzen.

Auch um den Einwirkbereich herum kann abschnittsweise eine Nut ausgebildet sein, welche wiederum bevorzugt das Anschlussbauteil in Dickenrichtung vollständig durchsetzt, um den Widerstand des Anschlussbauteils gegen eine Verlagerung des Einwirkbereichs zur Kopplungsachse hin oder/und von dieser weg zu verringern. Zum selben Zweck kann jener Bereich des Anschlussbauteils, welcher den Einwirkbereich umgibt, mit geringerer Materialstärke ausgebildet sein als der Einwirkbereich.

Bevorzugt weist das Anschlussbauteil mehr als einen Tragabschnitt auf, vorzugsweise genau zwei Tragabschnitte. Die zwei Tragabschnitte liegen einander bevorzugt die diametral bezüglich der Kopplungsachse gegenüber oder liegen wenigstens in einander diametral gegenüberliegenden Umfangssektoren mit je einer Winkelerstreckung von zwischen 20° und 75°. Dies ermöglicht die vorteilhafte einhändige Bedienung des Anschlussbauteils insbesondere beim Lösen von der Trachealkanüle, da die ein Einwirkbereiche von im Wesentlichen diametral gegenüberliegenden Tragabschnitten einander mit zwei Fingern einer Hand angenähert werden können, was gleichzeitig eine radiale Annäherung eines jeden dieser Einwirkbereiche an die Kopplungsachse bedeutet. Die Annäherung eines Einwirkbereichs eines Tragabschnitts an die Kopplungsachse bewirkt eine radiale Verlagerung der Verrastungsformation desselben Tragabschnitts.

Bevorzugt weist die Schürze zwei Schürzenabschnitte auf, zwischen welchen in Umfangsrichtung der wenigstens eine Tragabschnitt angeordnet ist. Durch Aufteilung der Schürze in zwei Schürzenabschnitte kann eine Schürze mit einer gewissen Verformbarkeit bereitgestellt werden, welche ein Verbinden des Anschlussbauteils mit der Trachealkanüle erleichtert. Zusätzlich oder alternativ kann der Kopplungsbereich die oben genannten bevorzugten zwei Tragabschnitte mit je einer Verrastungsformation aufweisen, wobei zwischen den Tragabschnitten in Umfangsrichtung je wenigstens ein Schürzenabschnitt angeordnet ist. So kann der Vorteil der einhändigen Bedienung mit dem Vorteil erhöhter Lagedefinition des Anschlussbauteils an der Trachealkanüle verbunden werden.

Um einerseits eine besonders sichere Verbindung des Anschlussbauteils mit der Trachealkanüle bereitstellen zu können und andererseits Verletzungen des Patienten durch das mit seiner Trachealkanüle gekoppelte Anschlussbauteil vermeiden zu können, kann der Kopplungsbereich gemäß einer bevorzugten Weiterbildung der vorliegenden Erfindung ein erstes, starreres Material aufweisen und kann das Anschlussbauteil an seinem dem Mündungsbereich nähergelegenen Abschnitt ein zweites, weniger starres Material aufweisen. Zur Beurteilung der Starrheit eines Materials wird sein Elastizitätsmodul herangezogen.

Auch ein solches, zwei unterschiedliche Werkstoffe umfassendes Anschlussbauteil kann durch Spritzgießen, nämlich durch Zwei-Komponenten-Spritzguss, erzeugt werden. Das Anschlussbauteil umfasst dann einen Bauteilhauptkörper aus zwei Komponenten: eine erste Bauteilhauptkörper-Komponente ist aus dem starreren Material gebildet und eine zweite, mit der ersten vorzugsweise stoffschlüssig verbundene, Bauteilhauptkörper-Komponente ist aus dem weniger starren Material gebildet.

Bevorzugt ist dann der Tragarm einstückig mit der ersten Bauteilhauptkörper-Komponente ausgebildet.

Um sicherzustellen, dass das Anschlussbauteil nicht ebenso unerwünscht durch einen Körperabschnitt des Patienten verschlossen wird, wie man das bei der Trachealkanüle befürchtet, kann das Anschlussbauteil am Mündungsbereich eine flächenmäßig größere Mündungsöffnung aufweisen und eine vom Mündungsbereich entfernt gelegene flächenmäßig kleinere Hilfsöffnung aufweisen. Selbst bei fehlerhafter Anordnung des Anschlussbauteils an einer Trachealkanüle und bei Anlage von Hautpartien des Patienten an das Anschlussbauteil bleibt wenigstens eine der Öffnungen aus Mündungsöffnung und Hilfsöffnung geöffnet. Bevorzugt befinden sich daher die Mündungsöffnung und die Hilfsöffnung bezüglich der Kopplungsachse in einander diametral gegenüberliegenden Umfangsabschnitten. Besonders bevorzugt liegen sich Mündungsöffnung und Hilfsöffnung bezüglich der Kopplungsachse diametral gegenüber.

Nachfolgend wird eine Beatmungsbaugruppe betrachtet, welche eine Trachealkanüle oder wenigstens ein Trachealkanülen-Endstück und ein mit der Trachealkanüle bzw. mit dem Trachealkanülen-Endstück koppelbares Anschlussbauteil, wie es oben beschrieben und weitergebildet ist, umfasst. Oben angegebene Ausgestaltungen des Anschlussbauteils und der Trachealkanüle im verbundenen Zustand sind Weiterbildungen dieser Beatmungsbaugruppe. Für die Verbindung des Anschlussbauteils mit der Trachealkanüle kommt es nur auf das Trachealkanülen-Endstück an, welches den zur Verbindung mit dem Anschlussbauteil benötigten Gegenkopplungsbereich aufweist. Bei Beschreibung eines mit einer Trachealkanüle bestimmungsgemäß verbundenen Anschlussbauteils, und nur ein solcher bestimmungsgemäßer Verbindungszustand ist oben überhaupt beschrieben, weist die Trachealkanüle stets ein Trachealkanülen-Endstück mit Gegenkopplungsbereich auf.

Das Trachealkanülen-Endstück weist folglich einen sich längs einer virtuellen Verbindungsachse erstreckenden Gegenkopplungsbereich mit der Verrastungsgegenformation auf zum formschlüssigen Verrastungseingriff mit der Verrastungsformation des Anschlussbauteils. Der Gegenkopplungsbereich umgibt einen von der Verbindungsachse zentral durchsetzt gedachten Kanülenhohlraum. Im bestimmungsgemäß verbundenen Zustand von Anschlussbauteil und Trachealkanülen-Endstück sind die Kopplungsachse und die Verbindungsachse kollinear oder parallel.

Die Verrastungsgegenformation kann in Umfangsrichtung vollständig um die Verbindungsachse umlaufen, erstreckt sich jedoch bevorzugt in Umfangsrichtung um die Verbindungsachse nur über einen Umfangsabschnitt des Gegenkopplungsbereichs, während ein weiterer Umfangsabschnitt nicht zum Verrastungseingriff mit der Verrastungsformation ausgebildet ist. Hierdurch kann die Anzahl möglicher Relativstellungen und damit die Anzahl möglicher unvorteilhafter Relativanordnungen von Anschlussbauteil und Trachealkanülen-Endstück begrenzt werden. Dort, wo ein Umfangsabschnitt nicht zum Verrastungseingriff mit der Verrastungsformation ausgebildet ist, kann das Anschlussbauteil für die es bedienende Bedienperson spürbar nicht verrasten, sodass eine etwaige Fehlanordnung oder wenigstens unvorteilhafte Anordnung des Anschlussbauteils relativ zum Trachealkanülen-Endstück bereits beim Ankoppeln des Anschlussbauteils an die Trachealkanüle korrigiert werden kann. Bei der genannten Relativstellung handelt es sich in der Regel um eine rotatorische Relativstellung des Anschlussbauteils relativ zum Trachealkanülen-Endstück in Umfangsrichtung um die Kopplungsachse bzw. Verbindungsachse.

Bevorzugt weist das Trachealkanülen-Endstück einen sich längs der Verbindungsachse erstreckenden Rohrabschnitt auf, an dessen einem Längsende bevorzugt der Gegenkopplungsbereich ausgebildet ist. Weiter kann das Trachealkanülen-Endstück eine vom Rohrabschnitt abstehende Verbindungsformation aufweisen, welche zur strömungsleitenden Verbindung mit einer Atemgasleitung ausgebildet ist. Somit kann das bevorzugt weitergebildete Trachealkanülen-Endstück sowohl mit dem Anschlussbauteil als auch mit einer Atemgasleitung verbunden werden. Durch die Verbindung mit der Atemgasleitung kann dem Patienten Atemgas im erforderlichen Ausmaß zugeführt werden.

Zur möglichst sicheren Vermeidung eines unerwünschten Verschlusses der Mündungsöffnung des Anschlussbauteils im bestimmungsgemäß an dem Trachealkanülen-Endstück angebrachten Zustand können die Verrastungsformation und die Verrastungsgegenformation derart an den jeweiligen Bauteilen angeordnet oder/und ausgebildet sein, dass eine Mündungsöffnung im Mündungsbereich des Anschlussbauteils im gleichen Umfangsabschnitt angeordnet ist wie die Verbindungsformation. In der Regel wird nämlich eine Atemgasleitung dem Trachealkanülen-Endstück aus einer Richtung zugeführt, in der möglichst wenig Wechselwirkung mit einem Körperteil des die Trachealkanüle tragenden Patienten zu erwarten ist. Dieser erwartete geringe Umfang an Wechselwirkung mit dem Patienten ist auch vorteilhaft für die Anordnung der Mündungsöffnung, sodass diese vorteilhaft in denselben Umfangsbereich öffnet, aus welchem dem Patienten Atemgas zugeführt wird.

Nicht immer ist die Körperhaltung eines beatmeten Patienten statisch oder liegt die dessen Körperhaltung von vornherein fest. Es ist daher vorteilhaft, das Anschlussbauteil mit einem gewissen Justagespiel am Trachealkanülen-Endstück anzuordnen, um auf eine unerwartete oder veränderte Körperhaltung des Patienten reagieren zu können. Da es für einen funktionierenden Schutz der Trachealkanüle vor einem Verschließen durch das Anschlussbauteil im Wesentlichen auf die Umfangsrichtung ankommt, in welche dessen Mündungsöffnung weist, kann das Anschlussbauteil im an dem Trachealkanülen-Endstück bestimmungsgemäß verrasteten, betriebsbereiten Zustand in einem Verschwenkbereich, der kleiner ist als der gesamte Umfang, relativ zum Trachealkanülen-Endstück um die Verbindungsachse verschwenkbar ausgebildet sein. Dies kann dadurch erreicht werden, dass ein Rastvorsprung aus Verrastungsformation und Verrastungsgegenformation in Umfangsrichtung kürzer ausgebildet ist als die mit dem Vorsprung zur Herstellung einer formschlüssigen Verrastung kooperierende Rastausnehmung.

Wie bereits eingangs dargelegt wurde, ist es zur dauerhaft sicheren Verbindung von Anschlussbauteil und Trachealkanüle vorteilhaft, wenn sich das Anschlussbauteil und das Trachealkanülen-Endstück im miteinander bestimmungsgemäß verrasteten, betriebsbereiten Zustand längs eines Überlappungsbereichs überlappen. Insbesondere können sich der Kopplungsbereich und der Gegenkopplungsbereich längs der dann kollinearen oder parallelen Achsen: Kopplungsachse und Verbindungsachse, überlappen. Die zur Betätigung des Einwirkbereichs benötigte Kraft kann dann betragsmäßig gering gehalten werden, wenn der Einwirkbereich außerhalb des Überlappungsbereichs gelegen ist.

Die vorliegende Erfindung wird nachfolgend anhand der beiliegenden Beschreibung näher erläutert werden. Es stellt dar:

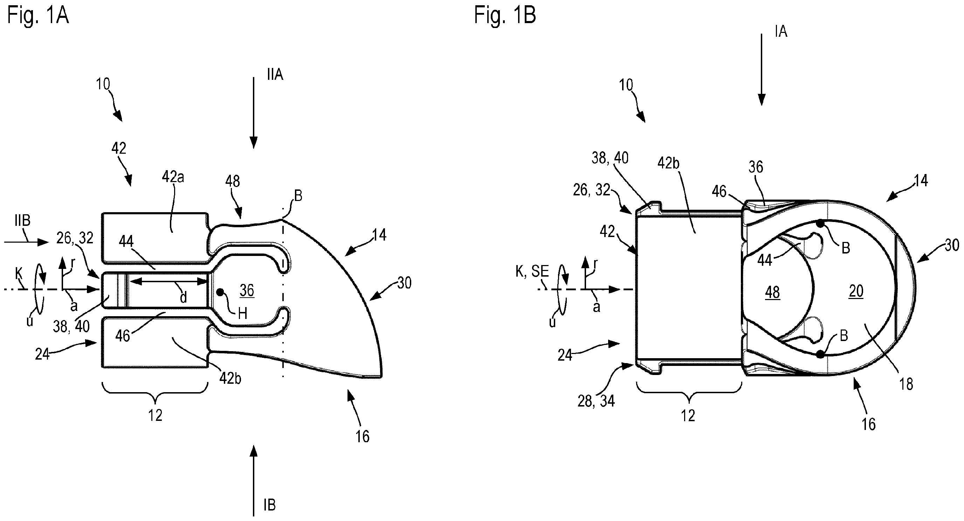

- Fig. 1A: eine Seitenansicht einer ersten Ausführungsform eines erfindungsgemäßen Anschlussbauteils mit einarmigem Tragarm,
- Fig. 1: B eine Unteransicht des Anschlussbauteils von Figur 1A, bei Betrachtung aus Richtung des Pfeils IB in Figur 1A,
- Fig. 2A: eine Draufsicht des Anschlussbauteils von Figur 1A, bei Betrachtung aus Richtung des Pfeils IIB in Figur 1A,
- Fig. 2B: eine Rückansicht des Anschlussbauteils von Figur 3A, bei Betrachtung aus Richtung des Pfeils IIB in Figur 1A,
- Fig. 3A: eine Seitenansicht einer zweiten Ausführungsform eines erfindungsgemäßen Anschlussbauteils mit zweiarmigem Tragarm,
- Fig. 3B: eine Rückansicht des Anschlussbauteils von Figur 3A, bei Betrachtung aus Richtung des Pfeils IIIB in Figur 3A,
- Fig. 4A: eine Seitenansicht einer dritten Ausführungsform eines erfindungsgemäßen Anschlussbauteils mit zweiarmigem Tragarm und mit unterschiedlich starren Materialien, die in unterschiedlichen Bereichen des Anschlussbauteils verwendet sind,
- Fig. 4B: eine Draufsicht des Anschlussbauteils von Figur 4A, bei Betrachtung aus Richtung des Pfeils IVB in Figur 4A,
- Fig. 5A: eine Seitenansicht einer ersten Ausführungsform einer erfindungsgemäßen Beatmungsbaugruppe, umfassend eine erste Ausführungsform eines Trachealkanülen-Endstücks und weiter umfassend das Anschlussbauteil der ersten Ausführungsform der Figuren 1A bis 2B,
- Fig. 5B: eine Unteransicht der Beatmungsbaugruppe von Figur 5A, bei Betrachtung Ausrichtung des Pfeils VB von Figur 5A,
- Fig. 6A: eine Seitenansicht einer zweiten Ausführungsform einer erfindungsgemäßen Beatmungsbaugruppe, umfassend eine zweite Ausführungsform eines Trachealkanülen-Endstücks und weiter umfassend das Anschlussbauteil der zweiten Ausführungsform der Figuren 3A und 3B, und
- Fig. 6B: eine Seitenansicht nur der zweiten Ausführungsform des Trachealkanülen-Endstücks von Figur 6A, ohne daran angekoppeltes Anschlussbauteil.

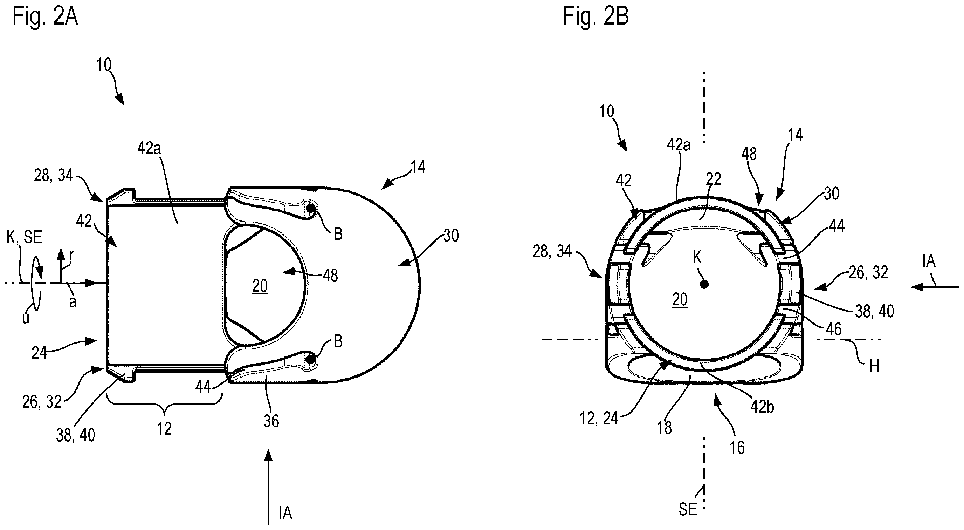

In den Figuren 1A bis 2B ist eine erste erfindungsgemäße Ausführungsform eines Anschlussbauteils allgemein mit 10 bezeichnet. In den Figuren 1B bis 2B geben die Pfeile IA die Blickrichtung von Figur 1A auf das Anschlussbauteil 10 an. Das Anschlussbauteil 10 weist einen sich längs einer Kopplungsachse K erstreckenden Kopplungsbereich 12 auf, welcher mit einem Hutzenabschnitt 14 einstückig verbunden ist. Der Hutzenabschnitt 14 weist einen Mündungsbereich 16 einer Mündungsöffnung 18 (siehe Figuren 1B und 2B) auf.

Die Kopplungsachse K definiert längs ihrer Verlaufsrichtung eine axiale Richtung a, orthogonal zu ihrer Verlaufsrichtung radiale Richtungen r und sie umgebend eine Umfangsrichtung u.

Die Mündungsöffnung 18 öffnet in radialer Richtung von der Kopplungsachse K weg.

Das Anschlussbauteil 10 umgibt einen Hohlraum 20, welcher sowohl von der Mündungsöffnung 18 aus als auch von einer Kopplungsöffnung 22 (siehe Figur 2B) aus, die an einem vom Hutzenabschnitt 14 abgewandten freien Kopplungsende 24 des Kopplungsbereichs 12 ausgebildet ist, von außerhalb des Anschlussbauteils 10 zugänglich ist.

Während der Kopplungsbereich 12 ausgehend von der Kopplungsöffnung 22 einen im Wesentlichen zylindrischen oder/und konischen Strömungskanalabschnitt längs der Kopplungsachse K bildet, bildet der Hutzenabschnitt 14 einen an den geradlinigen Strömungskanalabschnitt des Kopplungsbereichs 12 anschließenden, um eine zur Kopplungsachse K und zur Zeichenebene von Figur 1A orthogonale Krümmungsachse H gekrümmten Strömungskanalabschnitt aus, welcher zur Mündungsöffnung 18 führt.

Das Anschlussbauteil 10 umfasst zwei Tragabschnitte 26 und 28, welche als einseitig von einem Bauteilhauptkörper 30 auskragende Tragarme 32 bzw. 34 ausgebildet sind. Da das Anschlussbauteil 10 der ersten Ausführungsform bevorzugt spiegelsymmetrisch bezüglich einer die Kopplungsachse K enthaltenden, zur Zeichenebene der Figur 1A parallelen sowie zu den Zeichenebenen der Figuren 1B, 2A und 2B orthogonalen Spiegelsymmetrieebene SE ausgebildet ist, reicht es nachfolgend aus, nur den Tragabschnitt 26 bzw. den Tragarm 32 im Detail zu beschreiben. Die für den Tragabschnitt 26 und damit für den Tragarm 32 gegebene Beschreibung gilt unter der oben angegebenen Symmetriebedingung auch für den jeweils anderen Tragabschnitt 28 bzw. Tragarm 34.

Der als Tragarm 32 ausgebildete Tragabschnitt 26 ist einstückig mit dem Bauteilhauptkörper 30 ausgebildet und ist relativ zum Bauteilhauptkörper 30 um eine bevorzugt zur Kopplungsachse K orthogonale und mit Abstand von dieser verlaufende Biegeachse B biegbar. Um diese Biegung bewirken zu können, ist an einem der Biegeachse B näher gelegenen Abschnitt des Tragarms 32 ein Einwirkbereich 36 ausgebildet, auf welchen von außerhalb des Anschlussbauteils durch Hand- bzw. Fingerangriff eine bezüglich der Kopplungsachse K radial verlaufende, auf die Kopplungsachse K zu wirkende Betätigungskraft ausübbar ist. Der Einwirkbereich 36 ist Teil des Kopplungsbereichs 12.

Mit axialem Abstand d, jedoch ohne Umfangsabstand vom Einwirkbereich 36 ist einstückig mit dem Tragarm 32 und damit mit dem Einwirkbereich 36 eine Verrastungsformation 38 in Gestalt eines radial von der Kopplungsachse K weg vorstehenden Rastvorsprungs 40 ausgebildet. Durch Ausüben der zuvor beschriebenen Betätigungskraft auf den Einwirkbereich 36 kann die Verrastungsformation 38 gegen die Materialelastizität des Tragarms 32 und des Bauteilhauptkörpers 30 durch Biegeverformung um die Biegeachse B auf die Kopplungsachse K zu verlagert werden. Hierdurch kann die Verrastungsformation 38 aus einem bestehenden formschlüssigen Verrastungseingriff mit einer Verrastungsgegenformation einer Trachealkanüle bzw. eines Trachealkanülen-Endstücks genommen werden, um das Anschlussbauteil 10 von dem Trachealkanülen-Endstück zu lösen.

Der Kopplungsbereich 12 weist eine Schürze 42 mit zwei Schürzenabschnitten 42a und 42b auf, welche in Umfangsrichtung u um die Kopplungsachse K derart angeordnet sind, dass in Umfangsrichtung u auf einen Schürzenabschnitt ein Tragabschnitt bzw. ein Tragarm folgt und umgekehrt. Die Schürzenabschnitte 42a und 42b befinden sich im bestimmungsgemäß betriebsbereit an einem Trachealkanülen-Endstück angeordneten Zustand radial innerhalb eines Gegenkopplungsbereichs des Trachealkanülen-Endstücks und liegen diesem mit engem Radialspalt gegenüber.

Die Außenfläche der Schürzenabschnitte 42a und 42b sind bevorzugt teilzylindrisch oder teilkonisch mit der Kopplungsachse K als Zylinder- bzw. Konusachse ausgebildet, um eine gewisse Relativverdrehbarkeit des Anschlussbauteils 10 relativ zum Trachealkanülen-Endstück im bestimmungsgemäß mit dem Endstück gekoppelten Zustand gewährleisten zu können.

Zur Sicherstellung der Biegeverformbarkeit des Tragabschnitts 26 bzw. des Tragarms 32 ist dieser durch Nuten 44 und 46 in Umfangsrichtung beiderseits des Tragabschnitts 26 abschnittsweise vom Bauteilhauptkörper 30 getrennt.

Der Hutzenabschnitt 14 ist als gekrümmte Schale kuppelartig ausgebildet, um eine Verletzungsgefahr eines mit dem Hutzenabschnitt 14 in Kontakt tretenden Patienten, etwa durch Scheuern und dergleichen, zu vermeiden. Aus diesem Grunde sind auch die im Bereich des Hutzenabschnitt 14 gelegenen Einwirkbereiche (siehe Einwirkbereich 36) mit einer bei Betrachtung von außen konvex gekrümmten Oberfläche ausgebildet, welche über die Nuten 44 und 46 in Umfangsrichtung u hinweg eine stetige Fortsetzung der Oberfläche des übrigen Hutzenabschnitts 14 bildet. Der Hutzenabschnitt 14 und die Einwirkbereiche 36 weisen an ihrer Außenoberfläche keine Ecken und Kanten auf. Die Weite der Nuten 44 und 46 ist so gering, dass Nutränder keine Reizwirkung auf Haut eines Patienten ausüben können, die mit dem Hutzenabschnitt 14 in Kontakt treten. Im Übrigen können die Nutränder abgerundet ausgebildet sein.

Diametral der Mündungsöffnung 18 gegenüberliegend ist im Anschlussbauteil 10 eine Hilfsöffnung 48 ausgebildet, die allerdings wegen der hutzenartigen Ausbildung des Hutzenabschnitts 14 zwischen Kopplungsbereich 12 und Mündungsbereich 16 eine kleinere Öffnungsfläche aufweist als die Mündungsöffnung 18. Durch Ausbilden der Hilfsöffnung 18 kann jedoch selbst dann immer noch Atemgas zwischen der Hilfsöffnung 48 und der Kopplungsöffnung 22 strömen, wenn die Mündungsöffnung 18 unerwünschterweise bedeckt bzw. verschlossen ist. Da die Hilfsöffnung 48 der Mündungsöffnung 18 bezüglich der Kopplungsachse K diametral gegenüberliegt, ist es höchst unwahrscheinlich, dass beide Öffnungen 18 und 48 gleichzeitig durch unvorhergesehene Ereignisse verschlossen werden.

Der vom Anschlussbauteil 10 umgebene Hohlraum 20 verbindet im vorliegenden Ausführungsbeispiel die Öffnungen 18, 22 und 48 derart miteinander, dass jede dieser Öffnungen mit jeder anderen strömungsmechanisch kommunizieren kann.

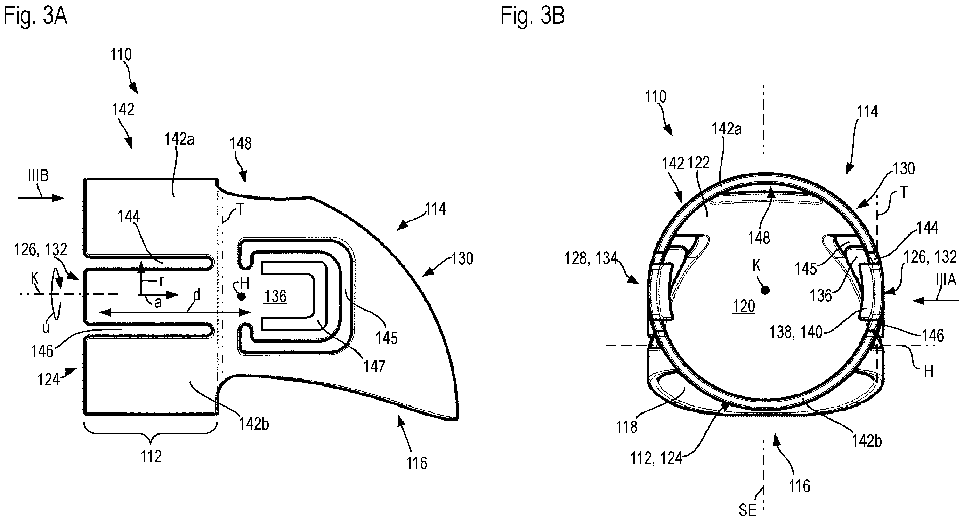

Als nächstes wird im Zusammenhang mit den Figuren 3A und 3B eine zweite Ausführungsform eines erfindungsgemäßen Anschlussbauteils 110 beschrieben werden. Gleiche und funktionsgleiche Bauteile und Bauteilabschnitte wie in der ersten Ausführungsform sind in der zweiten Ausführungsform mit gleichen Bezugszeichen versehen, jedoch erhöht um die Zahl 100. Die zweite Ausführungsform wird nachfolgend nur insofern beschrieben werden, als sie sich von der ersten Ausführungsform der Figuren 1A bis 2B unterscheidet, auf deren Beschreibung ansonsten auch zur Erläuterung der zweiten Ausführungsform ausdrücklich verwiesen wird. Der Pfeil IIIA in Figur 3B gibt die Richtung an, aus welcher das Anschlussbauteil 110 in Figur 3A betrachtet ist.

Der wesentliche Unterschied der zweiten Ausführungsform gegenüber der ersten Ausführungsform liegt darin, dass die als Tragarme 132 und 134 ausgebildeten Tragabschnitte 126 und 128 nicht als einarmige Hebel, sondern als zweiarmige Hebel ausgebildet sind. Wiederum wird mit Verweis auf die oben bereits beschriebene Spiegelsymmetrie nachfolgend stellvertretend für beide Tragabschnitte 126 und 128 nur der Tragabschnitt 126 beschrieben werden.

Der Tragabschnitt 126 bzw. der Tragarm 132 ist in einem Bereich zwischen dem Einwirkbereich 136 und der Verrastungsformation 138 in Umfangsrichtung u mit dem Bauteilhauptkörper 130 einstückig verbunden. Hierdurch sind die vom Kopplungsende 124 her axial in den Kopplungsbereich 112 hineinverlaufenden Nuten 144 und 146 axial kürzer ausgebildet als die entsprechenden Nuten 44 und 46 der ersten Ausführungsform, die durchgehend bis zur Verbindungsstelle des Einwirkbereichs 36 mit dem Bauteilhauptkörper 30 verlaufen. Durch die Anbindung des Tragabschnitts 126 an den Bauteilhauptkörper 130 zwischen Einwirkbereich 136 und Verrastungsformation 138 kann zur Erleichterung der verlagernden Betätigung des Einwirkbereichs 136 um den Einwirkbereich 136 herum eine weitere Nut 145 ausgebildet sein.

Alternativ zur Ausbildung der weiteren Nut 145 kann der Einwirkbereich 136 ohne Nut unterbrechungsfrei mit dem übrigen Bauteilhauptkörper 130 zusammenhängen. Eine verlagernde Betätigung des Einwirkbereichs 136 kann dann dadurch erleichtert sein, dass ein den Einwirkbereich 136 umgebender Bereich des Bauteilhauptkörpers 130 mit geringerer Materialdicke ausgebildet ist als der Einwirkbereich 136, so dass sich der den Einwirkbereich umgebende Umgebungsbereich mit geringerer Kraft verformen lässt als der Einwirkbereich 136, bzw. sich bei vorgegebener von außen auf die Kopplungsachse zu einwirkender Kraft stärker verformt als der Einwirkbereich 136. Folglich setzt der mit dem Einwirkbereich zusammenhängende Umgebungsbereich einer Verlagerung des Einwirkbereichs 136 nach radial innen auf die Kopplungsachse K zu nur einen unwesentlichen Widerstand entgegen.

Ganz grundsätzlich, aber insbesondere für den Fall, dass der Einwirkbereich nicht ohne Weiteres vom umgebenden übrigen Bauteilbereich unterscheidbar ist, etwa, weil keine Nut 145 vorhanden ist, ist es vorteilhaft, wenn am Einwirkbereich eine haptisch wahrnehmbare Formation vorgesehen ist, die den Ort des Einwirkbereichs ertastbar macht. Dann kann der Einwirkbereich auch bei Dunkelheit oder bei stark verschmutztem Anschlussbauteil aufgefunden werden. Eine solche Formation ist beispielhaft der U-förmige Vorsprung 147, welcher auf der von der Kopplungsachse K wegweisenden Außenseite des Anschlussbauteils 110 am Einwirkbereich 136 angeordnet ist. Vorzugsweise ist der nach radial außen vorstehende Vorsprung 147 einstückig mit dem Einwirkbereich 136 ausgebildet, etwa durch Spritzgießen.

Weiter bevorzugt ist der Vorsprung 147 ein längs der Kopplungsachse richtungsweisender Vorsprung 147, wie vorliegend beispielhaft ein U-förmiger Vorsprung 147, wobei bevorzugt die freien Schenkelenden längs der Kopplungsachse K zu der jeweiligen mit dem Einwirkbereich 136 am selben Tragabschnitt vorgesehenen Verrastungsformation 138, bzw. gekoppelten Zustand zum angekoppelten Trachealkanülen-Endstück weisen. Anstelle der gezeigten U-förmigen Gestalt kann eine andere Vorsprungsgestalt gewählt sein. Als richtungsweisende Vorsprungsgestalt kann beispielsweise ein Dreieck, ein Pfeil, eine V-, eine T- oder eine E-förmige Gestalt gewählt sein.

Im Gegensatz zur ersten Ausführungsform, bei welcher der Tragabschnitt 26 in axialer Richtung a einstückig mit dem Bauteilhauptkörper 30 verbunden ist, sodass Einwirkbereich 36 und Verrastungsformation 38 auf ein und derselben Seite der Biegeachse B gelegen sind, liegen bei der zweiten Ausführungsform der Einwirkbereich 136 und die Verrastungsformation 138 auf unterschiedlichen Seiten der Anbindung des Tragabschnitts 126 an den Bauteilhauptkörper 130.

In der Folge wird bei Ausübung einer Kraft auf den Einwirkbereich 136 in Richtung auf die Kopplungsachse K zu die Anbindung des Tragabschnitts 126 an den Bauteilhauptkörper nicht wie in der ersten Ausführungsform auf Biegung, sondern auf Torsion beansprucht. Bei Verlagerung des Einwirkbereichs 136 zur Kopplungsachse K hin tordiert die Anbindung des Tragabschnitts 126 gegen ihre Material- und Bauteilelastizität um die Torsionsachse T, wodurch die Verrastungsformation 138 zwar wiederum gleichzeitig mit dem Einwirkbereich 136 eine Verlagerung relativ zur Kopplungsachse K ausführt, die jedoch gegensinnig zur Verlagerung des Einwirkbereichs 136 ist. Die Verlagerung von Einwirkbereich 36 und Verrastungsformation 38 der ersten Ausführungsform sind dagegen gleichsinnig, d. h. entweder nähern sich beide genannten Bauteilabschnitte 36 und 38 gleichzeitig der Kopplungsachse K an oder es entfernen sich beide gleichzeitig von dieser.

Aufgrund der gegensinnigen Verlagerbarkeit von Einwirkbereich 136 und Verrastungsformation 138 ist der Kopplungsbereich 112 der zweiten Ausführungsform dazu ausgebildet, einen Gegenkopplungsbereich einer Trachealkanüle oder eines Trachealkanülen-Endstücks radial außen zu umgeben. Durch Verlagerung des Einwirkbereichs 136 zur Kopplungsachse K hin bzw. in den Hohlraum 120 hinein wird die Verrastungsformation 138 von der Kopplungsachse K weg verlagert und kann so beispielsweise aus einer Rastausnehmung am Außenumfang eines Gegenkopplungsbereichs verlagert werden.

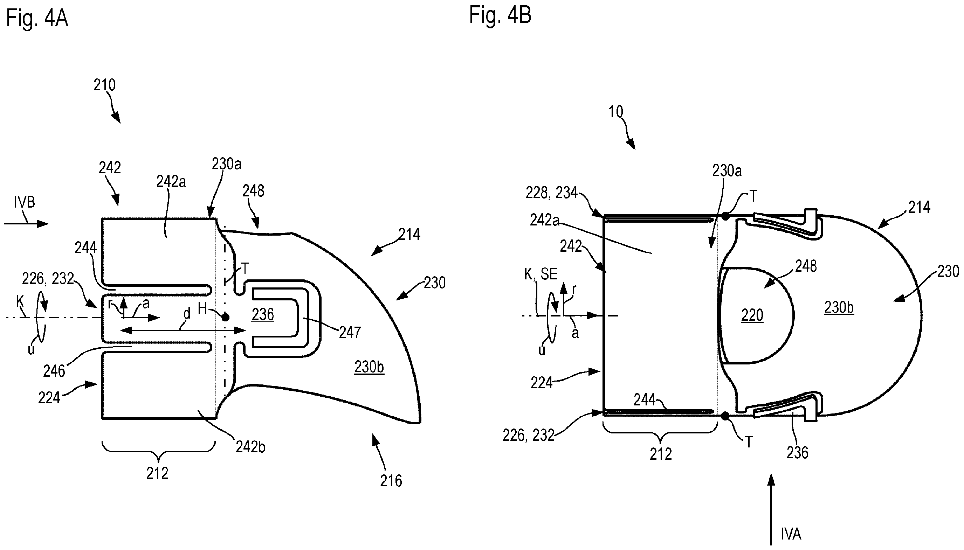

Als nächstes wird im Zusammenhang mit den Figuren 4A und 4B eine dritte Ausführungsform eines erfindungsgemäßen Anschlussbauteils 210 beschrieben werden. Gleiche und funktionsgleiche Bauteile und Bauteilabschnitte wie in der ersten oder/und zweiten Ausführungsform sind in der dritten Ausführungsform mit gleichen Bezugszeichen versehen, jedoch liegen diese zur Unterscheidung im Bereich von 200 bis 299. Die dritte Ausführungsform wird nachfolgend nur insofern beschrieben werden, als sie sich von der ersten und der zweiten Ausführungsform der Figuren 1A bis 3B unterscheidet, auf deren Beschreibung ansonsten auch zur Erläuterung der dritten Ausführungsform ausdrücklich verwiesen wird. Der Pfeil IVA in Figur 4B gibt die Richtung an, aus welcher das Anschlussbauteil 110 in Figur 3A betrachtet ist.

Die dritte Ausführungsform des Anschlussbauteils 210 entspricht in ihrer Funktion der zweiten Ausführungsform des Anschlussbauteils 110, d. h. die Tragabschnitte 226 und 228 sind als zweiarmige Hebel ausgebildet, welche um eine zwischen dem Einwirkbereich 236 und der Verrastungsformation 238 gelegenen Torsionsachse T drehen. Insofern funktioniert die dritte Ausführungsform hinsichtlich eines Herstellens und eines Lösens eines Formschlusseingriffs mit einer Verrastungsgegenformation wie die zweite Ausführungsform 110.

Die in den Figuren dargestellten Biegeachsen B und Torsionsachsen T sind Neigeachsen im Sinne der obigen Beschreibungseinleitung.

Die dritte Ausführungsform 210 ist aus zwei unterschiedlichen Materialien hergestellt, vorzugsweise in einem Zwei-Komponenten-Spritzgussverfahren. Der Bauteilhauptkörper 230 weist daher zwei Bauteilhauptkörper-Komponenten 230a und 230b auf.

Die erste Bauteilhauptkörper-Komponente 230a ist aus einem starreren Material gebildet, und umfasst im Wesentlichen den Kopplungsbereich 212. Die Tragabschnitte 226 und 228 sind vollständig aus demselben starreren Material gebildet wie die erste Bauteilhauptkörper-Komponente 230a und sind einstückig mit dieser ausgebildet.

Die zweite Bauteilhauptkörper-Komponente 230b ist aus einem weniger starren Material gebildet, beispielsweise aus einem thermoplastischen Elastomer, und umfasst im Wesentlichen den Hutzenabschnitt 214. Durch die Verwendung des weniger starren Materials im Hutzenabschnitt 214 gibt der Hutzenabschnitt 214 bei äußerer Belastung durch Verformung leichter nach als die starrere erste Bauteilhauptkörper-Komponente 230a, sodass der Hutzenabschnitt 214 selbst bei häufigerem Hautkontakt mit dem Patienten keine Irritationen und Verletzungen hervorruft. Im klinischen Einsatz kann der Hutzenabschnitt 114 in der Regel durch die Kinnpartie des Patienten erreichbar sein.

Zur Verbesserung der Verbindung von erster und zweiter Bauteilhauptkörper-Komponente 230a und 230b ist vorzugsweise zwischen dem Rand des Einwirkbereichs 236 und der zweiten Bauteilhauptkörper-Komponente 230b keine Nut ausgebildet. Stattdessen ist die zweite Bauteilhauptkörper-Komponente 230b unmittelbar an den Einwirkbereich 236 angespritzt. Wegen des geringeren Elastizitätsmoduls des Materials der zweiten Bauteilhauptkörper-Komponente 230b ist das Anspritzen dieser Komponente 230b an den Einwirkbereich 236 für die Beweglichkeit des Einwirkbereichs 236 unschädlich. Die zur Verlagerung des Einwirkbereichs 236 auf die Kopplungsachse K zu benötigte Kraft ist im Vergleich zur zweiten Ausführungsform durch das Anspritzen der zweiten Bauteilhauptkörper-Komponente 230b nur unwesentlich erhöht.

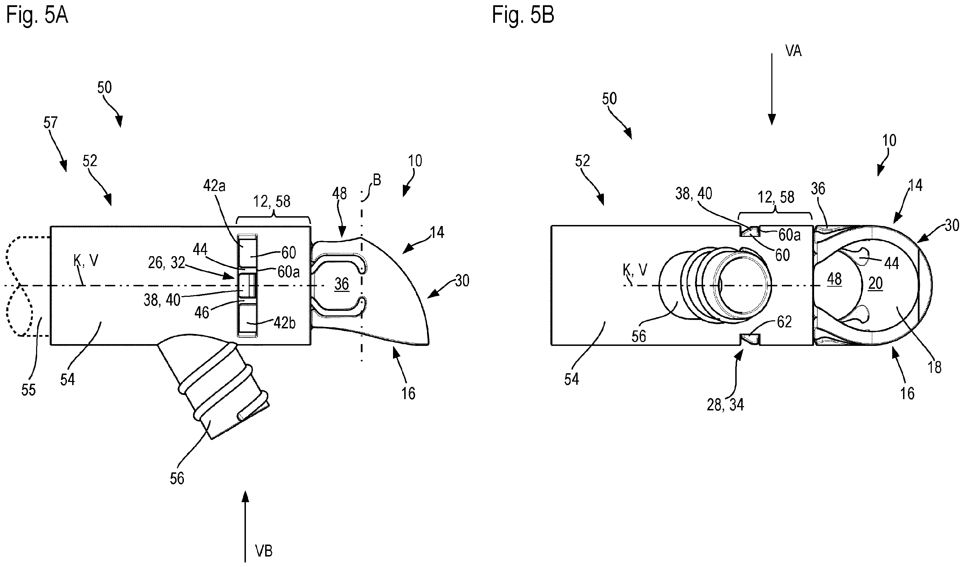

In den Figuren 5A und 5B ist eine erste Ausführungsform einer erfindungsgemäßen Beatmungsbaugruppe 50 gezeigt. Figur 5A zeigt die Beatmungsbaugruppe 50 von der Seite, Figur 5B von unten. Die Betrachtungsrichtung von Figur 5A ist in Figur 5B durch den Pfeil VA angegeben.

Die Beatmungsbaugruppe 50 umfasst ein Anschlussbauteil 10 gemäß den Figuren 1A bis 2B, welches bestimmungsgemäß mit einem Trachealkanülen-Endstück 52 gekoppelt ist. Das Trachealkanülen-Endstück 52 umfasst einen Rohrabschnitt 54, welcher im dargestellten Beispiel im Wesentlichen zylindrische Gestalt hat, jedenfalls bevorzugt geradlinig entlang der verlängert gedachten Kopplungsachse K verläuft. An seinem vom Anschlussbauteil 10 fernliegenden Längsende ist das Trachealkanülen-Endstück 52 in an sich bekannter Weise mit einem nur strichliniert angedeuteten Trachealkanülenkanal 55 zur Bildung einer Trachealkanüle 57 verbunden oder verbindbar.

An seiner Unterseite steht vom Rohrabschnitt 54 ein Verbindungsstutzen 56 ab, welcher zur Verbindung mit einer nicht dargestellten Atemgasleitung ausgebildet ist. Durch den Verbindungsstutzen 56 als Verbindungsformation zur Verbindung mit einer Atemgasleitung kann einem Patienten über die Trachealkanüle 57 erforderlichenfalls Atemgas verabreicht werden.

Das Anschlussbauteil ist bevorzugt derart mit dem Trachealkanülen-Endstück 52 gekoppelt, dass die Mündungsöffnung 18 in die Richtung weist, in welcher der Verbindungsstutzen 56 vom Rohrabschnitt 54 absteht. Da in der klinischen Anwendung der Verbindungsstutzen 56 in der Regel vom Kopf des die Trachealkanüle 57 tragenden Patienten wegweisend angeordnet ist, weist die Mündungsöffnung 18 des gemäß Figur 5A mit dem Trachealkanülen-Endstück 52 gekoppelten Anschlussbauteils ebenfalls vom Kopf des Patienten weg, sodass die Mündungsöffnung 18 durch den Patienten selbst bei sehr unvorteilhafter Körperhaltung nicht erreichbar und somit nicht verschließbar ist.

Das dem Anschlussbauteil 10 nächstgelegene Längsende des Rohrabschnitts 54 ist als Gegenkopplungsbereich 58 ausgebildet. Der Gegenkopplungsbereich 58 und der Kopplungsbereich 12 überlappen sich bei bestimmungsgemäßer Kopplung des Anschlussbauteils 10 mit dem Trachealkanülen-Endstück 52. Die zuvor beschriebenen Schürzenabschnitte 42a und 42b liegen mit einem engen Radialspalt Innenflächen des Gegenkopplungsbereichs 58 gegenüber. Der Abstand d ist so bemessen, dass sich der Einwirkbereich 36 mit dem Gegenkopplungsbereich 58 nicht überlappt. Er ist in seiner Betätigbarkeit daher nicht durch den Gegenkopplungsbereich 58 behindert.

Der Gegenkopplungsbereich 58 erstreckt sich längs einer Verbindungsachse V, welche in dem dargestellten bestimmungsgemäßen Kupplungszustand mit der Kopplungsachse K des Kopplungsbereichs 12 des Anschlussbauteils 10 kollinear ist.

Der Gegenkopplungsbereich 58 umfasst an zwei bezüglich der Verbindungsachse V einander diametral gegenüberliegenden Umfangsabschnitten je eine den Rohrabschnitt 54 in Dickenrichtung vollständig durchsetzende Rastausnehmung 60 und 62 als Verrastungsgegenformationen. In die Rastausnehmung 60 greift der Rastvorsprung 40 des Tragabschnitts 26 bzw. Tragarms 32 formschlüssig ein, in die Rastausnehmung 62 der Rastvorsprung des Tragabschnitts 28 bzw. Tragarms 34. Die Schürzenabschnitte 42a und 42b sowie die Endbereiche der Tragarme bedecken die Rastausnehmung 60 und verschließen diese zu einem Großteil.

Der Rastvorsprung 40 hintergreift dabei verrastend einen dem Anschlussbauteil 10 näher liegenden Rand 60a der Rastausnehmung 60 und verhindert so ein Ausziehen des Anschlussbauteils 10 aus dem Trachealkanülen-Endstück 52. Ein Ausziehen ist jedoch möglich, wenn die Einwirkbereiche 36 der Tragabschnitte 26 und 28 soweit zur Kopplungsachse K hin verlagert werden, dass die Verrastungsformation 38 des Tragabschnitts 26 und die Verrastungsformation des Tragabschnitts 28 außer Eingriff mit den hintergriffenen Kanten der Rastausnehmungen 60 bzw. 62 geraten.

Die Rastausnehmungen 60 und 62 verlaufen jeweils nur über einen vorbestimmten Winkelbereich in Umfangsrichtung u um die Kopplungsachse K bzw. um die Verbindungsachse V. Längs ihrer Umfangserstreckung gestatten die Rastausnehmungen 60 und 62 eine Relativbewegung, genauer eine Relativverdrehung des Anschlussbauteils 10 relativ zum Rohrabschnitt 54 um die Kopplungsachse K. Dadurch kann in den vorgegebenen Grenzen das Anschlussbauteil 10 und insbesondere die Mündungsöffnung 18 situationsgerecht justiert werden.

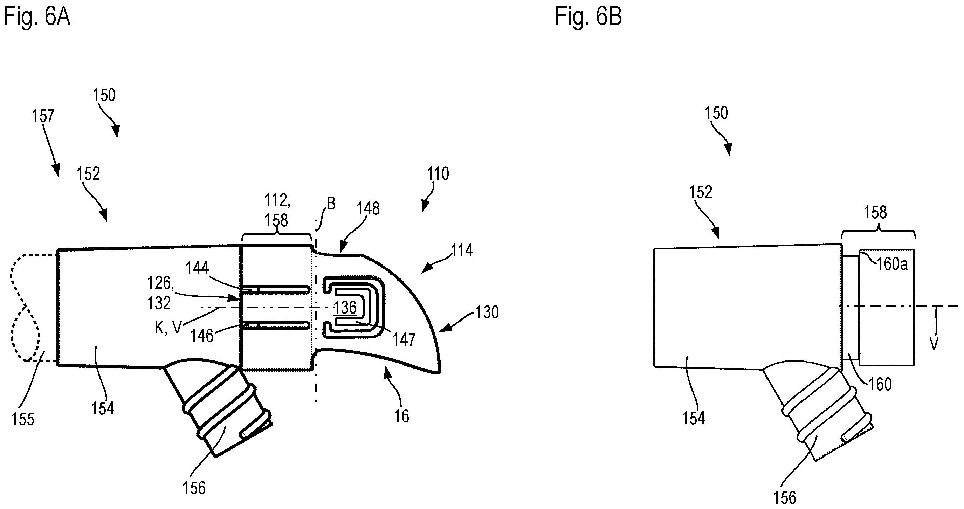

In Figur 6A ist eine zweite Ausführungsform einer erfindungsgemäßen Beatmungsbaugruppe 150 gezeigt, welche eine zweite Ausführungsform eines Trachealkanülen-Endstücks 152 und daran bestimmungsgemäß angekoppelt die zweite Ausführungsform des Anschlussbauteils 110 der Figuren 3A und 3B zeigt.

Gleiche und funktionsgleiche Bauteile und Bauteilabschnitte wie in der ersten Ausführungsform der Beatmungsbaugruppe 50 der Figuren 5A und 5B sind in der zweiten Ausführungsform von Figur 6A mit gleichen Bezugszeichen versehen, jedoch erhöht um die Zahl 100. Die zweite Ausführungsform der Beatmungsbaugruppe 150 wird nachfolgend nur insofern beschrieben werden, als sich von der ersten Ausführungsform unterscheidet, auf deren Beschreibung ansonsten auch zur Erläuterung der zweiten Ausführungsform verwiesen wird.

Der im Wesentlichen einzige funktionale Unterschied zwischen der zweiten und der ersten Ausführungsform der Beatmungsbaugruppe liegt darin, dass das Anschlussbauteil 110 mit seinem Kopplungsbereich 112 den Gegenkopplungsbereich 158 des Trachealkanülen-Endstück 152 bezüglich der kollinearen Achsen: Kopplungsachse K und Verbindungsachse V radial außen umgibt.

Die Verrastungsformationen der Tragabschnitte 126 und 128, also beispielsweise der Rastvorsprung 140, greifen folglich nicht von radial innen nach radial außen in eine Verrastungsgegenformation des Gegenkopplungsbereichs 158 ein, sondern in entgegengesetzter Richtung von radial außen nach radial innen.

Die Verrastungsgegenformation des Trachealkanülen-Endstück 152 ist als vollständig umlaufende Nut 160 ausgebildet, kann jedoch auch nur in Umfangsrichtung bereichsweise am Rohrabschnitt 154 ausgebildet sein. Die Nut 160 durchsetzt den Rohrabschnitt 154 in Dickenrichtung nicht.

Da der Kopplungsbereich 112 den Gegenkopplungsbereich 158 in Figur 6A im angekoppelten Zustand bei Betrachtung von radial außen verdeckt, ist in Figur 6B das Trachealkanülen-Endstück 152 von Figur 6A in Alleinstellung ohne Anschlussbauteil 110 dargestellt. Zu erkennen ist die umlaufende Nut 160 als Rastausnehmung und deren von den Verrastungsformationen der Tragabschnitte 126 und 128 hintergriffener Rand 160a, welcher einen kleineren Durchmesser aufweist als die Außenfläche des übrigen Rohrabschnitts jenseits der Nut 160. Wegen des kleineren Durchmessers des Gegenkopplungsbereichs 158, verglichen mit dem übrigen Rohrabschnitt, kann das Anschlussbauteil 110 im angekoppelten Zustand eine im Wesentlichen stufenlose, bündige Außenfläche längs der gemeinsamen Achsen: Kopplungsachse K und Verbindungsachse V über im Wesentlichen die gesamte Außenfläche der Beatmungsbaugruppe 150 mit Ausnahme des Hutzenabschnitts 114 und des Verbindungsstutzens 156 bilden.

Anstelle des Anschlussbauteils 110 kann auch das Anschlussbauteil 210 an das Trachealkanülen-Endstück 152 angekoppelt werden.

## Patentansprüche

1. Anschlussbauteil (10; 110; 210) für eine Trachealkanüle (57; 157), wobei das Anschlussbauteil (10; 110; 210) einen Hohlraum (20; 120; 220) umgibt, wobei das Anschlussbauteil (10; 110; 210) sowohl an einem Kopplungsbereich (12; 112; 212) des Anschlussbauteils (10; 110; 210) als auch an einem vom Kopplungsbereich (12; 112; 212) örtlich verschiedenen Mündungsbereich (16; 116; 216) des Anschlussbauteils (10; 110; 210) zur Bereitstellung eines Zugangs zu dem Hohlraum (20; 120; 220) offen oder öffenbar ist, wobei das Anschlussbauteil (10; 110; 210) am Kopplungsbereich (12; 112; 212) eine Verrastungsformation (38; 138; 238) aufweist, welche zur lösbaren Verbindung mit einer Verrastungsgegenformation (60, 62; 162) der Trachealkanüle (57; 157) ausgebildet ist, wobei der Kopplungsbereich (12; 112; 212) einen Abschnitt des Hohlraums umgibt, wobei der von dem Kopplungsbereich (12; 112; 212) umgebene Abschnitt des Hohlraums (20; 120; 220) zentral von einer virtuellen Kopplungsachse (K) durchsetzt gedacht ist, wobei die Kopplungsachse (K) längs ihres Verlaufs eine axiale Richtung (a), orthogonal zu ihrem Verlauf eine Vielzahl von radialen Richtungen (r) und um sie umlaufend eine Umfangsrichtung (u) definiert, wobei der Kopplungsbereich (12; 112; 212) wenigstens einen Tragabschnitt (26, 28; 126, 128; 226, 228) aufweist, an welchem an einem ersten Ort die Verrastungsformation (38; 138; 238) und an einem vom ersten Ort verschiedenen zweiten Ort ein Einwirkbereich (36; 136; 236) derart angeordnet sind, dass durch Ausübung einer Betätigungskraft auf den Einwirkbereich (36; 136; 236) in radialer Richtung auf die Kopplungsachse (K) zu der Tragabschnitt (26, 28; 126, 128; 226, 228) verlagerbar ist zwischen zwei Stellungen unterschiedlichen radialen Abstands der am Tragabschnitt (26, 28; 126, 128; 226, 228) angeordneten Verrastungsformation (38; 138; 238) von der Kopplungsachse (K), wobei der Einwirkbereich (36; 136; 236) und die durch Kraftausübung auf den Einwirkbereich (36; 136; 236) verlagerbare Verrastungsformation (38; 138; 238) desselben Tragabschnitts (26, 28; 126, 128; 226, 228) längs der Kopplungsachse (K) mit axialem Abstand (d) voneinander angeordnet sind,
**dadurch gekennzeichnet, dass** das Anschlussbauteil (10; 110; 210) zwischen dem Kopplungsbereich (12; 112; 212) und dem Mündungsbereich (16; 116; 216) als um eine zur Kopplungsachse (K) orthogonale Krümmungsachse (H) gekrümmtes Rohr oder gekrümmte Hutze ausgebildet ist.

2. Anschlussbauteil (10; 110; 210) nach Anspruch 1,
wobei der Einwirkbereich (36; 136; 236) und die Verrastungsformation (38) desselben Tragabschnitts (26, 28; 126, 128; 226, 228) in Umfangsrichtung (u) um die Kopplungsachse (K) wenigstens abschnittsweise, vorzugsweise vollständig, im gleichen Umfangsbereich angeordnet sind.

3. Anschlussbauteil (10; 110; 210) nach Anspruch 1 oder 2,
wobei der Tragabschnitt (26, 28; 126, 128; 226, 228) einen mit einem Bauteilhauptkörper (30; 130; 230) relativ zu diesem beweglich verbundenen Tragarm (32, 34; 132, 134; 232, 234) umfasst, der durch Ausübung der Betätigungskraft um eine zur Kopplungsachse (K) windschiefe Neigeachse (B; T) neigbar ist, wobei die Erstreckungsrichtung der Neigeachse (B; T) mit der Erstreckungsrichtung der Kopplungsachse (K) einen Winkel im Bereich von 70° bis 110° einschließt.

4. Anschlussbauteil (10) nach Anspruch 3,
wobei der Einwirkbereich (36) und die Verrastungsformation (38) auf derselben Seite der Neigeachse (B) am selben Tragabschnitt (26, 28) gelegen sind, sodass eine radiale Betätigungsverlagerung des Einwirkbereichs (36) eine gleichsinnige, aber betragsmäßig von der Betätigungsverlagerung verschiedene Wirkverlagerung der Verrastungsformation (38) desselben Tragabschnitts (26, 28) bewirkt.

5. Anschlussbauteil (10) nach Anspruch 4,
wobei der Einwirkbereich (36) näher an der Neigeachse (B) gelegen ist als die zugeordnete Verrastungsformation (38), sodass der Tragabschnitt (26, 28) die Betätigungsverlagerung in eine betragsmäßig größere Wirkverlagerung übersetzt.

6. Anschlussbauteil (110; 210) nach Anspruch 3,
wobei der Einwirkbereich (136; 236) und die Verrastungsformation (138; 238) auf unterschiedlichen Seiten der Neigeachse (T) am selben Tragabschnitt (126, 128; 226, 228) gelegen sind, sodass eine radiale Betätigungsverlagerung des Einwirkbereichs (136; 236) eine gegensinnige Wirkverlagerung der Verrastungsformation (138; 238) desselben Tragabschnitts (126, 128; 226, 228) bewirkt.

7. Anschlussbauteil (10; 110; 210) nach einem der Ansprüche 3 bis 6,
wobei der Tragarm (32, 34; 132, 134; 232, 234) einstückig mit dem Bauteilhauptkörper (30; 130; 230) verbunden ist, so dass eine Verlagerung der Verrastungsformation (38; 138; 238) durch Verformung wenigstens eines Abschnitts des Anschlussbauteils (10; 110; 210) bewirkt ist, wobei die Neigeachse (B; T) eine Biegeachse (B) oder/und eine Torsionsachse (T) ist.

8. Anschlussbauteil (10; 110; 210) nach einem der vorhergehenden Ansprüche,
wobei der Kopplungsbereich (12; 112; 212) eine Schürze (42; 142; 242) aufweist, welche die Kopplungsachse (K) längs eines Winkelbereichs umgibt, wobei der Tragabschnitt (26, 28; 126, 128; 226, 228) von der Schürze (42; 142; 242) durch eine Nut (44, 46; 144, 146; 244, 246) getrennt ist.

9. Anschlussbauteil (10; 110; 210) nach Anspruch 8,
wobei die Schürze (42; 142; 242) zwei Schürzenabschnitte (42a, 42b; 142a, 142b; 242a, 242b) aufweist, zwischen welchen in Umfangsrichtung (u) der Tragabschnitt (26, 28; 126, 128; 226, 228) angeordnet ist, oder/und dass der Kopplungsbereich (12; 112; 212) zwei Tragabschnitte (26, 28; 126, 128; 226, 228) mit je einer Verrastungsformation (38; 138; 238) aufweist, zwischen welchen in Umfangsrichtung je wenigstens ein Schürzenabschnitt (42a, 42b; 142a, 142b; 242a, 242b) angeordnet ist.

10. Anschlussbauteil (210) nach einem der vorhergehenden Ansprüche,
wobei der Kopplungsbereich (212) ein erstes, starreres Material aufweist und dass das Anschlussbauteil (210) an seinem dem Mündungsbereich (216) nähergelegenen Abschnitt ein zweites, weniger starres Material aufweist.

11. Anschlussbauteil (10; 110; 210) nach einem der vorhergehenden Ansprüche,
wobei das Anschlussbauteil (10; 110; 210) am Mündungsbereich (16; 116; 216) eine flächenmäßig größere Mündungsöffnung (18; 118; 218) aufweist und eine vom Mündungsbereich (16; 116; 216) entfernt gelegene flächenmäßig kleinere Hilfsöffnung (48; 148; 248) aufweist.

12. Beatmungsbaugruppe (50; 150), umfassend ein Trachealkanülen-Endstück (52; 152) und ein mit diesem koppelbares Anschlussbauteil (10; 110; 210) nach einem der vorhergehenden Ansprüche,
wobei das Trachealkanülen-Endstück (52; 152) einen sich längs einer Verbindungsachse (V) erstreckenden Gegenkopplungsbereich (58; 158) mit der Verrastungsgegenformation (60, 62; 160) zum formschlüssigen Verrastungseingriff mit der Verrastungsformation (38; 138; 238) des Anschlussbauteils (10; 110; 210) aufweist, wobei der Gegenkopplungsbereich (58; 158) einen von der Verbindungsachse (V) zentral durchsetzt gedachten Kanülenhohlraum umgibt, wobei sich bevorzugt die Verrastungsgegenformation (60, 62) in Umfangsrichtung (u) um die Verbindungsachse (V) nur über einen Umfangsabschnitt des Gegenkopplungsbereichs (58) erstreckt, während ein weiterer Umfangsabschnitt nicht zum Verrastungseingriff mit der Verrastungsformation (38; 138; 238) ausgebildet ist.

13. Beatmungsbaugruppe (50; 150) nach Anspruch 12,
wobei das Trachealkanülen-Endstück (52; 152) einen sich längs der Verbindungsachse (V) erstreckenden Rohrabschnitt (54; 154) und eine vom Rohrabschnitt (54; 154) abstehende Verbindungsformation (56; 156) aufweist, welche zur strömungsleitenden Verbindung mit einer Atemgasleitung ausgebildet ist, wobei die Verrastungsformation (38; 138; 238) und die Verrastungsgegenformation (60, 62; 160) derart an den jeweiligen Bauteilen angeordnet oder/und ausgebildet sind, dass eine Mündungsöffnung (18; 118; 218) im Mündungsbereich (16; 116; 216) des Anschlussbauteils (10; 110; 210) im an dem Trachealkanülen-Endstück (52; 152) verrasteten, betriebsbereiten Zustand im gleichen Umfangsabschnitt angeordnet ist wie die Verbindungsformation (56; 156).

14. Beatmungsbaugruppe nach Anspruch 12 oder 13,
wobei das Anschlussbauteil (10; 110; 210) im an dem Trachealkanülen-Endstück (52; 152) verrasteten, betriebsbereiten Zustand in einem Verschwenkbereich, der kleiner ist als der gesamte Umfang, relativ zum Trachealkanülen-Endstück (52; 152) um die Verbindungsachse (V) verschwenkbar ist.

15. Beatmungsbaugruppe nach einem der Ansprüche 12 bis 14,
wobei sich das Anschlussbauteil (10; 110; 210) und das Trachealkanülen-Endstück (52; 152) im miteinander verrasteten, betriebsbereiten Zustand längs eines Überlappungsbereichs (12, 58; 112; 158) überlappen, wobei der Einwirkbereich (36; 136; 236) außerhalb des Überlappungsbereichs (12, 58; 112; 158) gelegen ist.

## Claims

1. An attachment (10; 110; 210) for a tracheal cannula (57; 157), the attachment (10; 110; 210) surrounding a cavity (20; 120; 220), the attachment (10; 110; 210) being open or openable both in a coupling area (12; 112; 212) of the attachment (10; 110; 210) as well as in an orifice area (16; 116; 216) of the attachment (10; 110; 210), differing in location from the coupling area (12; 112; 212), for providing an access to the cavity (20; 120; 220), the attachment (10; 110; 210) having in the coupling area (12; 112; 212) a latching formation (38; 138; 238), which is designed for a releasable connection to a mating latching formation (60, 62; 162) of the tracheal cannula (57; 157), the coupling area (12; 112; 212) surrounding a section of the cavity, the section of the cavity (20; 120; 220) surrounded by the coupling area (12; 112; 212) being centrally penetrated by virtual coupling axis (K), the coupling axis (K) defining an axial direction (a) along its path, defining a plurality of radial directions (r) orthogonally to its path and defining a circumferential direction (u) running around it, the coupling area (12; 112; 212) having at least one support section (26, 28; 126, 128; 226, 228), on which at a first location the latching formation (38; 138; 238) is situated and at a second location distinct from the first location a force application area (36; 136; 236) is situated in such a way that by exerting an actuating force on the force application area (36; 136; 236) in the radial direction toward the coupling axis (K), the support section (26, 28; 126, 128; 226, 228) is displaceable between two positions of different radial distance of the latching formation (38; 138; 238) situated on the support section (26, 28; 126, 128; 226, 228) from the coupling axis (K),
wherein the force application area (36; 136; 236) and the latching formation (38; 138; 238) displaceable by an exertion of force on the force application area (36; 136; 236) of the same support section (26, 28; 126, 128; 226, 228) are situated along the coupling axis (K) at an axial distance (d) from each other,
**characterized in that** the attachment (10; 110; 210) is formed between the coupling area (12; 112; 212) and the orifice area (16; 116; 216) as a tube or a hood curved around an axis of curvature (H) that is orthogonal to the coupling axis (K).

2. The attachment (10; 110; 210) as recited in Claim 1,
wherein the force application area (36; 136; 236) and the latching formation (38) of the same support section (26, 28; 126, 128; 226, 228) are situated in the circumferential direction (u) around the coupling axis (K) at least in sections, preferably entirely, in the same circumferential area.

3. The attachment (10; 110; 210) as recited in Claim 1 or 2,
wherein the support section (26, 28; 126, 128; 226, 228) comprises a support arm (32, 34; 132, 134; 232, 234) connected to a main component body (30; 130; 230) in a manner that is movable relative to the latter, which, by exerting the actuating force, is tiltable around a tilting axis (B; T) that is skewed with respect to the coupling axis (K), the extension direction of the tilting axis (B; T) together with the extension direction of the coupling axis (K) enclosing an angle in the range of 70° to 110°.

4. The attachment (10) as recited in Claim 3,
wherein the force application area (36) and the latching formation (38) are situated on the same side of the tilting axis (B) on the same support section (26, 28), so that a radial actuation displacement of the force application area (36) effects an effective displacement of the latching formation (38) of the same support section (26, 28) in the same direction, but of different magnitude.

5. The attachment (10) as recited in Claim 4,
wherein the force application area (36) is situated closer to the tilting axis (B) than the associated latching formation (38), so that the support section (26, 28) transforms the actuation displacement into an effective displacement that is greater in terms of magnitude.

6. The attachment (110; 210) as recited in Claim 3,
wherein the force application area (136; 236) and the latching formation (138, 238) are situated on different sides of the tilting axis (T) on the same support section (126, 128; 226, 228), so that a radial actuation displacement of the force application area (136; 236) effects an oppositely directed effective displacement of the latching formation (138; 238) of the same support section (126, 128; 226, 228).

7. The attachment (10; 110; 210) as recited in one of Claims 3 through 6,
wherein the support arm (32, 34; 132, 134; 232, 234) is connected in one piece with the main component body (30; 130; 230), so that a displacement of the latching formation (38; 138; 238) is effected by deformation of at least one section of the attachment (10; 110; 210), the tilting axis (B; T) being a bending axis (B) and/or a torsion axis (T).

8. The attachment (10; 110; 210) as recited in one of the preceding claims,
wherein the coupling area (12; 112; 212) includes an apron (42; 142; 242), which surrounds the coupling axis (K) along an angular range, the support section (26, 28; 126, 128; 226, 228) being separated from the apron (42; 142; 242) by a groove (44, 46; 144, 146; 244, 246).

9. The attachment (10; 110; 210) as recited in Claim 8,
wherein the apron (42; 142; 242) has two apron sections (42a, 42b; 142a, 142b; 242a, 242b), between which in the circumferential direction (u), the support section (26, 28; 126, 128; 226, 228) is situated, and/or the coupling area (12; 112; 212) includes two support sections (26, 28; 126, 128; 226, 228) having each one latching formation (38; 138; 238), between which in the circumferential direction respectively at least one apron section (42a, 42b; 142a, 142b; 242a, 242b) is situated.

10. The attachment (210) as recited in one of the preceding claims,
wherein the coupling area (212) comprises a first, more rigid material and the attachment (210) comprises in its section closer to the orifice area (216) a second, less rigid material.

11. The attachment (10; 110; 210) as recited in one of the preceding claims,
wherein the attachment (10; 110; 210) has at the orifice area (16; 116; 216) a greater orifice opening (18; 118; 218) in terms of area and a smaller auxiliary opening (48; 148; 248) in terms of area that is remote from the orifice area (16; 116; 216).

12. A respiratory assembly (50; 150), comprising a tracheal cannula end piece (52; 152) and an attachment (10; 110; 210) couplable to the latter as recited in one of the preceding claims,
wherein the tracheal cannula end piece (52; 152) has a mating coupling area (58; 158) extending along a connection axis (V) including the mating latching formation (60, 62; 160) for the form-locking engagement with the latching formation (38; 138; 238) of the attachment (10; 110; 210), the mating coupling area (58; 158) surrounding a cannula cavity centrally virtually penetrated by the connection axis (V), the mating latching formation (60, 62) preferably extending in the circumferential direction (u) around the connection axis (V) only over a circumferential section of the mating coupling area (58), while a further circumferential section is not designed for the latching engagement with the latching formation (38; 138; 238).

13. The respiratory assembly (50; 150) as recited in Claim 12,
wherein the tracheal cannula end piece (52; 152) includes a tube section (54; 154) extending along the connection axis (V) and a connection formation (56; 156) protruding from the tube section (54, 154), which is formed for the flow-conducting connection to a respiratory gas line, the latching formation (38; 138; 238) and the mating latching formation (60, 62; 160) being situated and/or formed on the respective components in such a way that an orifice opening (18; 118; 218) in the orifice area (16; 116; 216) of the attachment (10; 110; 210) is situated in the operational state latched on the tracheal cannula end piece (52; 152) in the same circumferential section as the connection formation (56; 156).

14. The respiratory assembly as recited in Claim 12 or 13,
wherein the attachment (10; 110; 210), in the operational state latched on the tracheal cannula end piece (52; 152), is swivable relative to the tracheal cannula end piece (52; 152) around the connection axis (V) in a swivel range that is smaller than the entire circumference.

15. The respiratory assembly as recited in one of Claims 12 through 14,
wherein the attachment (10; 110; 210) and the tracheal cannula end piece (52; 152) overlap in the mutually latched operational state along an overlap area (12, 58; 112; 158), the force application area (36; 136; 236) being situated outside of the overlap area (12, 58; 112; 158).

## Revendications

1. Composant de raccordement (10; 110; 210) pour une canule trachéale (57; 157), dans lequel le composant de raccordement (10; 110; 210) entoure une cavité (20; 120; 220), dans lequel le composant de raccordement (10; 110; 210) est ouvert ou peut être ouvert aussi bien à une zone d'accouplement (12; 112; 212) du composant de raccordement (10; 110; 210) ainsi qu'à une zone d'embouchure (16; 116; 216) du composant de raccordement (10; 110; 210) localement différente de la zone d'accouplement (12; 112; 212) pour fournir un accès à la cavité (20; 120; 220), dans lequel le composant de raccordement (10; 110; 210) présente dans la zone d'accouplement (12; 112; 212) une formation d'encliquetage (38; 138; 238) qui est configuré pour être reliée de manière amovible à une contre-formation d'encliquetage (60, 62; 162) de la canule trachéale (57; 157), dans lequel la zone d'accouplement (12; 112; 212) entoure une partie de la cavité, dans lequel la partie de la cavité (20; 120; 220) entouré par la zone d'accouplement (12; 112; 212) est pensée traversée au centre par un axe de couplage virtuel (K), dans lequel l'axe d'accouplement (K) définit le long de son tracé une direction axiale (a), orthogonalement à son tracé une pluralité de directions radiales (r) et autour de lui une direction périphérique (u), dans lequel la zone d'accouplement (12; 112; 212) présente au moins une section de support (26, 28; 126, 128; 226, 228) sur laquelle sont disposées, à un premier endroit, la formation d'encliquetage (38; 138; 238) et, à un deuxième endroit différent du premier endroit, une zone d'action (36; 136; 236) de telle sorte que, par l'exercice d'une force d'actionnement sur la zone d'action (36; 136; 236) dans la direction radiale vers l'axe d'accouplement (K), la section de support (26, 28; 126, 128; 226, 228) peut être déplacée entre deux positions de distance radiale différente de la formation d'encliquetage (38; 138, 238) disposée sur la section de support (26, 28; 126, 128; 226, 228) de l'axe d'accouplement (K), dans lequel la zone d'action (36; 136; 236) et la formation d'encliquetage (38; 138; 238) de la même section de support (26, 28; 126, 128; 226, 228) pouvant être déplacée par l'exercice d'une force sur la zone d'action (36; 136; 236) sont disposées le long de l'axe d'accouplement (K) à une distance axiale (d) l'une de l'autre,
**caractérisé en ce que** le composant de raccordement (10; 110; 210) est réalisé entre la zone d'accouplement (12; 112; 212) et la zone d'embouchure (16; 116; 216) sous la forme d'un tube ou d'un chapeau courbé autour d'un axe de courbure (H) orthogonal à l'axe d'accouplement (K).

2. Composant de raccordement (10; 110; 210) selon la revendication 1, dans lequel la zone d'action (36; 136; 236) et la formation d'encliquetage (38) de la même section de support (26, 28; 126, 128; 226, 228) sont disposées dans la direction circonférentielle (u) autour de l'axe d'accouplement (K) au moins par sections, de préférence entièrement, dans la même zone circonférentielle.

3. Composant de raccordement (10; 110; 210) selon la revendication 1 ou 2, dans lequel la partie de support (26, 28; 126, 128; 226, 228) comprend un bras de support (32, 34; 132, 134; 232, 234) raccordé de manière mobile à un corps principal de composant (30; 130; 230) qui, par l'exercice de la force d'actionnement, peut être incliné autour d'un axe d'inclinaison (B; T) décalé angulairement par rapport à l'axe d'accouplement (K), dans lequel la direction d'extension de l'axe d'inclinaison (B; T) forme avec la direction d'extension de l'axe d'accouplement (K) un angle dans la plage de 70° à 110°.

4. Composant de raccordement (10) selon la revendication 3, dans lequel la zone d'action (36) et la formation d'encliquetage (38) sont situées du même côté de l'axe d'inclinaison (B) sur la même section de support (26, 28), de sorte qu'un déplacement d'actionnement radial de la zone d'action (36) provoque un déplacement actif de même sens, mais différent en valeur absolue du déplacement d'actionnement, de la formation d'encliquetage (38) de la même section de support (26, 28).

5. Composant de raccordement (10) selon la revendication 4, dans lequel la zone d'action (36) est située plus près de l'axe d'inclinaison (B) que la formation d'encliquetage (38) associée, de sorte que la section de support (26, 28) transforme le déplacement d'actionnement en un déplacement d'action plus important en valeur absolue.

6. Composant de raccordement (110; 210) selon la revendication 3, dans lequel la zone d'action (136; 236) et la formation d'encliquetage (138; 238) sont situées sur des côtés différents de l'axe d'inclinaison (T) sur la même section de support (126, 128; 226, 228), de sorte qu'un déplacement d'actionnement radial de la zone d'action (136; 236) provoque un déplacement d'action de sens opposé de la formation d'encliquetage (138; 238) de la même section de support (126, 128; 226, 228).

7. Composant de raccordement (10; 110; 210) selon l'une des revendications 3 à 6, dans lequel le bras de support (32, 34; 132, 134; 232, 234) est relié d'un seul tenant au corps principal de composant (30; 130; 230), de sorte qu'un déplacement de la formation d'encliquetage (38; 138; 238) est provoqué par déformation d'au moins une section du composant de raccordement (10; 110; 210), dans lequel l'axe d'inclinaison (B; T) est un axe de flexion (B) ou/et un axe de torsion (T).

8. Composant de raccordement (10; 110; 210) selon l'une des revendications précédentes, dans lequel la zone d'accouplement (12; 112; 212) comporte une jupe (42; 142; 242) entourant l'axe d'accouplement (K) selon une plage angulaire, dans lequel la portion de support (26, 28; 126, 128; 226, 228) est séparée de la jupe (42; 142; 242) par une rainure (44, 46; 144, 146; 244, 246).

9. Composant de raccordement (10; 110; 210) selon la revendication 8, dans lequel la jupe (42; 142; 242) présente deux sections de jupe (42a, 42b; 142a, 142b; 242a, 242b) entre lesquelles se trouve, dans la direction périphérique (u), la section de support (26, 28; 126, 128; 226, 228), ou/et en ce que la zone d'accouplement (12; 112; 212) présente deux sections de support (26, 28; 126, 128; 226, 228) avec chacune une formation d'encliquetage (38; 138; 238), entre lesquelles est disposée dans la direction périphérique au moins une section de jupe (42a, 42b; 142a, 142b; 242a, 242b).

10. Composant de raccordement (210) selon l'une des revendications précédentes, dans lequel la zone de couplage (212) présente un premier matériau plus rigide et en ce que le composant de raccordement (210) présente un deuxième matériau moins rigide sur sa portion la plus proche de la zone d'embouchure (216).

11. Composant de raccordement (10; 110; 210) selon l'une des revendications précédentes, dans lequel le composant de raccordement (10; 110; 210) présente, au niveau de la zone d'embouchure (16; 116; 216), une ouverture d'embouchure (18; 118; 218) de plus grande surface et présente une ouverture auxiliaire (48; 148; 248) de plus petite surface, éloignée de la zone d'embouchure (16; 116; 216).

12. Ensemble de ventilation (50; 150) comprenant un embout de canule trachéale (52; 152) et un composant de raccordement (10; 110; 210) pouvant être couplé à celui-ci selon l'une des revendications précédentes, dans lequel l'embout de canule trachéale (52; 152) comporte une zone de contre-couplage (58; 158) s'étendant le long d'un axe de liaison (V) avec la contre-formation d'encliquetage (60, 62; 160) pour un engagement d'encliquetage par complémentarité de forme avec la formation d'encliquetage (38; 138; 238) du composant de raccordement (10; 110; 210), DANS LEQUEL la zone de contre-accouplement (58; 158) entoure une cavité de canule pensée traversée centralement par l'axe de liaison (V), dans lequel la contre-formation d'encliquetage (60, 62) s'étend de préférence dans la direction périphérique (u) autour de l'axe de liaison (V) uniquement sur une section périphérique de la zone de contre-accouplement (58), tandis qu'une autre section périphérique n'est pas réalisée pour l'engagement d' encliquetage avec la formation d'encliquetage (38; 138; 238).

13. Ensemble de ventilation (50; 150) selon la revendication 12, dans lequel l'embout de canule trachéale (52; 152) comprend une section de tube (54; 154) s'étendant le long de l'axe de connexion (V) et une formation de connexion (56; 156) faisant sailli à partir de la section de tube (54; 154), qui est conçue pour une connexion de guidage d'écoulement avec une conduite de gaz respiratoire, dans lequel la formation d'encliquetage (38; 138; 238) et la contre-formation d'encliquetage (60, 62; 160) sont disposées ou/et réalisées sur les composants respectifs de telle sorte qu'une ouverture d'embouchure (18; 118; 218) dans la zone d'embouchure (16; 116; 216) du composant de raccordement (10; 110; 210) est disposée dans la même section périphérique que la formation de liaison (56; 156) dans l'état encliqueté prêt à l'emploi sur la pièce d'extrémité de canule trachéale (52; 152).

14. Ensemble de ventilation selon la revendication 12 ou 13, dans lequel le composant de raccordement (10; 110; 210) peut pivoter par rapport à l'embout de canule trachéale (52; 152) autour de l'axe de liaison (V) dans une zone de pivotement inférieure à l'ensemble de la périphérie, lorsqu'il est à l'état encliqueté sur l'embout de canule trachéale (52; 152) et prêt à fonctionner.

15. Ensemble de ventilation selon l'une des revendications 12 à 14, dans lequel le composant de raccordement (10; 110; 210) et l'embout de canule trachéale (52; 152) se chevauchent le long d'une zone de chevauchement (12, 58; 112; 158) lorsqu'ils sont enclenchés l'un avec l'autre et prêts à fonctionner, dans lequel la zone d'action (36; 136; 236) est située en dehors de la zone de chevauchement (12, 58; 112; 158).
